(19) Europäisches Patentamt
European Patent Office
Office européen des brevets

(11) EP 4 110 478 B1

(12) # EUROPEAN PATENT SPECIFICATION

(45) Date of publication and mention of the grant of the patent:
**01.01.2025 Bulletin 2025/01**

(21) Application number: **21714185.2**

(22) Date of filing: **26.03.2021**

(51) International Patent Classification (IPC):
***A61Q 19/10*** (2006.01) ***A61K 8/34*** (2006.01)
***A61K 8/41*** (2006.01) ***A61K 8/39*** (2006.01)
***A61K 8/44*** (2006.01) ***A61Q 17/00*** (2006.01)

(52) Cooperative Patent Classification (CPC):
**A61K 8/34; A61K 8/347; A61K 8/39; A61K 8/416; A61K 8/442; A61Q 17/005; A61Q 19/10;**
A61K 2800/70

(86) International application number:
**PCT/EP2021/057890**

(87) International publication number:
**WO 2021/191413 (30.09.2021 Gazette 2021/39)**

(54) **DISINFECTANT COMPOSITIONS**

DESINFIZIERENDE ZUSAMMENSETZUNGEN

COMPOSITIONS DÉSINFECTANTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.03.2020 EP 20166072**
**23.12.2020 EP 20217019**

(43) Date of publication of application:
**04.01.2023 Bulletin 2023/01**

(73) Proprietor: **Arch UK Biocides Ltd**
**Manchester, M9 8GQ (GB)**

(72) Inventors:
- **CLARKE, James**
  **Castleford, West Yorkshire WF10 2JT (GB)**
- **SHAW, Neil Scott**
  **Castleford, West Yorkshire WF10 2JT (GB)**
- **PAMBOU, Elias**
  **Castleford, West Yorkshire WF10 2JT (GB)**
- **NIGHTINGALE, Aaron Thomas Arnold**
  **Castleford, West Yorkshire WF10 2JT (GB)**
- **JACKSON, Rachel Emily**
  **Castleford, West Yorkshire WF10 2JT (GB)**
- **WILLETT, James David**
  **Castleford, West Yorkshire WF10 2JT (GB)**

(74) Representative: **Michalski Hüttermann & Partner Patentanwälte mbB**
**Kaistraße 16A**
**40221 Düsseldorf (DE)**

(56) References cited:
**WO-A1-01/41727**
**WO-A1-2019/142194**
**WO-A1-97/23594**
**CN-A- 109 362 768**
**CN-A- 110 897 914**
**CN-A- 111 096 340**
**US-A1- 2002 022 660**
**US-A1- 2003 022 941**
**US-A1- 2008 103 210**
**US-A1- 2012 201 902**
**US-A1- 2018 153 177**
**US-A1- 2019 125 634**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 110 478 B1

## Description

### TECHNICAL FIELD

The present disclosure relates to antimicrobial skin disinfectant compositions such as hand disinfectant soaps, comprising one or more quaternary ammonium compounds, one or more surfactants and an aromatic alcohol.

### BACKGROUND

Disinfection is required in several industries because of both regulatory and safety concerns. Industries that require employees to consistently maintain anti-microbial disinfection include the industrial and institutional (I & I) cleaning sector and health services such as hospitals, doctors' offices, and EMT units. Additionally, the food industry requires strict disinfection procedures for employees working in restaurants and in factories that prepare and package food items. To maintain disinfection, workers may need to disinfect an area of their body regularly throughout a workday. For example, a nurse working at a hospital may be required to wash her hands in between each visit to a new patient, which could lead to 50 or more hand washings in a single day. Many antimicrobial handwashing compositions are known in the art. WO 2017/184614 A1 discloses antimicrobial compositions comprising among other components a cationic antimicrobial agent (e.g. benzalkonium chloride), a surfactant or a surfactant combination, and a foam stabilizer. WO 2013/148247 A1 discloses antimicrobial compositions comprising a cationic antimicrobial agent selected from the group consisting of lauric arginate and benzalkonium chloride, one or more non-ionic surfactants, an amine oxide and one or more preservatives. US 2002/0022660 relates to antimicrobial compositions comprising antimicrobial components and a combination of surfactants that do not include anionic surfactants. US 2019/0125634 discloses a foaming antimicrobial composition, which includes a cationic antimicrobial, a combination of surfactants, skin conditioning agent(s), humectant(s), and water. US 2018/0153177 relates to antimicrobial compositions comprising mixtures of botanical extracts, synthetic antimicrobial agents and essential oils. CN 110897914 discloses a foam hand sanitizer compositions comprising among others a foaming agent, benzalkonium chloride, glycerin, polysorbate-20, and phenoxyethanol. CN 111096340 relates to cleaning and disinfecting articles comprising among others phenoxyethanol. However, broad concentration ranges for each of the components are disclosed both in WO 2017/184614 A1 and in WO 2013/148247 A and none of the prior art citations has documented specific concentration ranges for each of the components in terms of obtaining compositions free from dermal or eye irritation with adequate bactericidal and yeasticidal efficacy and aesthetic benefits.

Thus, there is a need of finding out the perfect balance between the right components and the right concentrations of the different components of the antimicrobial composition in order to get a composition free from dermal or eye irritation that at the same time, delivers the necessary foaming aesthetics and dermatological benefits (i.e. moisturisation), and is identified as bacteriacidal and yeasticidal even in dirty medical conditions at low contact times (e.g. 60 s or less).

### SUMMARY

The problem underlying the present invention has been solved by a skin disinfectant composition comprising:

a. one or more quaternary ammonium compounds in an amount of from 0.1 to 2.0 wt% based upon the total weight of the composition, wherein said one or more quaternary ammonium compounds comprise a dimethyl dialkyl ammonium chloride wherein each alkyl group contains 8 to 12 carbon atoms;
b. one or more surfactants in an amount of from 0.1 to 4.0 wt% based upon the total weight of the composition, wherein the one or more surfactants are selected from the group consisting of non-ionic surfactants, amphoteric surfactants, and mixtures thereof, wherein the non-ionic surfactant is selected from the group consisting of polyglyceryl-10 caprylate/caprate, polysorbate 20, polysorbate 80, decyl glucoside, polyethylene glycol (23) lauryl ether, C9-11 hexaethylene glycol alkyl ethers and polyoxyethylene (21) stearyl ether, and wherein
the amphoteric surfactant is selected from the group consisting of octyldimethyl amine oxide, decyldimethyl amine oxide, lauryldimethyl amine oxide, isoalkyl dimethyl amine oxide, tetradecyldimethyl amine oxide, cetyldimethyl amine oxide, cocoamidopropyl betaine and ß-Alanine, N-(2-carboxyethyl)-, N-coco alkyl derivatives; and
c. an aromatic alcohol in an amount of from 0.1 to 1.0 wt% based upon the total weight of the composition, wherein the aromatic alcohol is phenoxyethanol, and
wherein the composition does not contain an anionic surfactant.

In certain embodiments, the skin disinfectant composition may pass the European standard testing protocol EN13727 in 60 s or less contact time and may pass the European standard testing protocol EN13624 in 60 s or less contact time. In one embodiment, the skin disinfectant composition may pass the European standard testing protocol EN13727 in 30 s or less contact time and may pass the European standard testing protocol EN13624 in 30 s or less

contact time.

The composition does not contain an anionic surfactant.

The amount of one or more surfactants in the skin disinfectant composition is of 0.1 to 4.0 wt% based upon the total weight of the composition.

In certain embodiments, the one or more surfactants present in the skin disinfectant composition may comprise at least a non-ionic surfactant selected from the group consisting of polyglyceryl-10 caprylate/caprate, polysorbate 20, polysorbate 80, decyl glucoside, polyethylene glycol (23) lauryl ether, C9-11 hexaethylene glycol alkyl ethers and polyoxyethylene (21) stearyl ether.

In certain embodiments, the one or more surfactants may comprise at least an amphoteric surfactant selected from the group consisting of octyldimethyl amine oxide, decyldimethyl amine oxide, lauryldimethyl amine oxide, isoalkyl dimethyl amine oxide, tetradecyldimethyl amine oxide, cetyldimethyl amine oxide, cocoamidopropyl betaine and ß-Alanine, N-(2-carboxyethyl)-, N-coco alkyl derivatives.

In certain embodiments, the compositions may comprise an aromatic alcohol in an amount of from 0.1 to 1.0 wt% based upon the total weight of the composition and at least one amphoteric surfactant in an amount of from 0.1 to 4.0 wt%, based upon the total weight of the composition.

In some of the embodiments, the compositions may comprise at least a non-ionic surfactant and at least an amphoteric surfactant.

In some of the embodiments, the one or more quaternary ammonium compounds may be present in an amount of 0.2 to 1.0 wt% based on the total weight of the composition. In other embodiments, the one or more quaternary ammonium compounds may be present in an amount of 0.2 to 0.6 wt% based on the total weight of the composition.

Disclosed are skin disinfectant compositions, wherein the aromatic alcohol may be present in an amount of from 0.1 to 1.0 wt% based upon the total weight of the composition, the one or more surfactants may comprise at least a non-ionic surfactant and at least an amphoteric surfactant in an amount of from 1.0 to 6.0 wt% based upon the total weight of the composition, and the weight ratio of amphoteric surfactant to non-ionic surfactant may be 1:1 to 1:6.

In certain embodiments, the aromatic alcohol may be present in an amount of from 0.1 to 0.5 wt% based upon the total weight of the composition, the one or more surfactants may comprise at least a non-ionic surfactant and at least an amphoteric surfactant in an amount of from 1.0 to 4.0 wt% based upon the total weight of the composition and the weight ratio of amphoteric surfactant to non-ionic surfactant may be 1:1 to 1:6.

In some embodiment, the one or more quaternary ammonium compounds is didecyldimethylammonium chloride.

Disclosed are skin disinfectant compositions, wherein the one or more quaternary ammonium compounds are selected from the group consisting of didecyldimethylammonium chloride, benzalkonium chloride and mixtures thereof.

In some embodiment, the one or more surfactants are selected from the group consisting of polysorbate 20, lauryl dimethyl amine oxide, polyglyceryl-10 caprylate/caprate, and mixtures thereof.

The aromatic alcohol is phenoxyethanol.

In some embodiment, the composition according to the present invention comprises

a. didecyldimethylammonium chloride in an amount of from 0.1 to 0.7 wt.%, based upon the total weight of the composition;
b. one or more surfactants selected from polysorbate 20, lauryl dimethyl amine oxide, and polyglyceryl-10 caprylate/caprate in an amount of from 0.3 to 3.0 wt%, based upon the total weight of the composition; and
c. 2-phenoxyethanol in an amount of from 0.1 to 1.0 wt%, based upon the total weight of the composition.

In some embodiment, the composition may further comprise a humectant. In certain embodiments, the humectant may be selected from the group consisting of propylene glycol, glycerol, diglycerol, triglycerol, hexaglycerol and triglycerol.

In certain embodiments, the composition may further comprise a solvent. In certain embodiments, the solvent is water.

In any of the embodiments, the composition may further comprise at least one additive selected from the group consisting of fragrances, colouring agents or dyes, opacifying agents, pearlizing agents, vitamins, antioxidants, emollients, skin care additives, polymeric thickeners, and foaming agents.

In any of the embodiments the composition may further comprise a humectant, a solvent, a fragrance, a colouring agent or dye, an opacifying agent, a pearlizing agent, a vitamin, an antioxidant, an emollient, a skin care additive, a foaming agent, a polymeric thickener or mixtures thereof.

In another aspect, there is provided a hand soap in the form of a liquid, gel or foam comprising, consisting essentially or consisting of the skin disinfectant composition of the present disclosure. Other features and aspects of the present disclosure are set forth in greater detail below.

## DETAILED DESCRIPTION

### Definitions

As used herein, reference to an element by the indefinite article "a" or "an" does not exclude the possibility that more than one of the element is present, unless the context clearly requires that there is one and only one of the elements. The indefinite article "a" or "an" thus usually means "at least one." The disclosure of numerical ranges should be understood as referring to each discrete point within the range, inclusive of endpoints, unless otherwise noted. The term "about" as used in the disclosure of numerical ranges indicates that deviation from the stated value is acceptable to the extent that the deviation is the result of measurement variability and/or yields a product of the same or similar properties.
"In an amount" when referring to two or more ingredients (e.g. surfactants) should be understood as the sum of the amounts of each individual ingredient.
The term "comprising", "comprises" and "comprised of" as used herein are synonymous with "including", "includes" or "containing", "contains", and are inclusive or open-ended and do not exclude additional, non-recited members, elements, components or method steps. The terms also encompass "consisting of" and "consisting essentially of".
Unless otherwise specified, all terms used in disclosing the invention, including technical and scientific terms, have the meaning as commonly understood by one of ordinary skill in the art to which this invention belongs.

### Testing of the compositions

In order for a skin disinfectant composition to be considered an effective anti-microbial sanitizer or disinfectant, it should be able to demonstrate efficacy against a specific range of different microorganisms including as gram-positive, gram-negative types of bacteria and yeast. A product or composition is considered antibacterial if it shows efficacy against selected gram-positive and gram-negative bacterial. A product or composition is considered yeasticidal if is shows efficacy against selected yeast. A product or composition is considered antimicrobial if it shows antimicrobial efficacy against selected gram-positive bacteria, gram-negative bacterial and yeast. In addition to bacteria and yeast, in certain manifestations the disinfectant composition of the present disclosure may also kill and control the growth of various other microorganisms, such as viruses, particularly enveloped viruses,
**[0030]** Examples of particular microorganisms that may be controlled in accordance with the present disclosure include *staphylococcus aureus, Streptococcus pneumoniae, pseudomonas aeruginosa, Serratia marcescens, Salmonella enteritidis, Neisseria gonorrhoeae, Escherichia coli, Enterococcus hirae, Acinetobacter baumannii, Listeria monocytogenes, Enterobacter gergoviae, Klebsiella pneumoniae, Burholderia cepacia, pseudomonas putida, Kocuria rhizophila, Candida albicans, Coronavirus, Adenovirus, Norovirus, Vaccinia virus, Influenza virus, Hepatitis 8 virus, Human Immunodeficiency virus, Human papilloma virus,* or mixtures thereof.
Generally, antimicrobial efficacy can be determined using a standard set of tests designed to investigate the antimicrobial properties of a said product or composition when used in a defined application. The testing of said compositions is required to ensure that the products are effective when used in hygiene or disinfectant applications in disinfecting microorganisms including bacteria and yeast. Standard test protocols such as ASTM and EN methods (amongst other method standards) are designed to replicate the use conditions of the products to be tested against a set of organisms, designed to show that the product will perform in said application.
The required tests are adapted to the specific efficacy to be measured and to the area of application. For example for industrial and institutional cleaning and food and beverage applications the European standard testing protocol EN1276 specifies the minimum requirements for bactericidal activity of chemical disinfectant and antiseptic products that form a homogeneous, physically stable preparation when diluted with hard water or - in the case of ready-to-use products - with water; and European standard testing protocol EN1650 is a test method, which specifies the minimum requirements for fungicidal or yeasticidal activity of chemical disinfectant and antiseptic products that form a homogeneous, physically stable preparation when diluted with hard water or - in the case of ready-to-use-products with water.
For applications in industrial and institutional cleaning and food and beverage applications and in the medical area, the European standard testing protocol EN13624 specifies a test method and the minimum requirements for fungicidal or yeasticidal activity of chemical disinfectant and antiseptic products that form a homogeneous, physically stable preparation when diluted with hard water, or - in the case of ready-to-use products - with water and European standard testing protocol EN13727 specifies the minimum requirements for bactericidal activity of chemical disinfectant and antiseptic products that form a homogeneous, physically stable preparation when diluted with hard water, or - in the case of ready-to-use products - with water.
A composition demonstrating antimicrobial efficacy in medical dirty conditions, according to EN13727 and EN13624 can be assumed to have antimicrobial efficacy according to the European testing norms EN1276 and EN1650. By demonstrating antimicrobial efficacy according to EN13727 and EN13624 the tested product or composition meets

the minimum requirements for fungicidal or yeasticidal activity of chemical disinfectant and antiseptic products in industrial and institutional cleaning and food and beverage applications and in the medical area.

In the European test norms antimicrobial efficacy studies for hand disinfectant products are carried on a specified range of different of bacteria and yeast strains:

*Candida albicans* - yeast (EN13624).
*Enterococcus hirae* - gram positive (EN13727).
*staphylococcus aureus* - gram positive (EN13727).
*pseudomonas aeruginosa* - gram negative (EN13727).
*Escherichia coli* - (EN13727, EN1499).

The skin disinfectant compositions of the disclosure may pass the European standard testing protocol EN13727 in 60 s or less contact time and may pass the European standard testing protocol EN13624 in 60 s or less contact time. In certain embodiments, the skin disinfectant compositions of the disclosure may pass the European standard testing protocol EN13727 in 30 s or less contact time and may pass the European standard testing protocol EN13624 in 30 s or less contact time. In other embodiments, the skin disinfectant compositions of the disclosure may pass the European standard testing protocol EN13727 in 15 s or less contact time and may pass the European standard testing protocol EN13624 in 15 s or less contact time.

As the compositions of the disclosure can pass in 60 s or less contact time, such as 30 s or less contact time or 15 s or less contact time, the European standard testing protocol EN13727, they can be described as bacteriacidal. They can be regarded as fungicidal or yeasticidal as they can pass in about 60 s or less contact time, such as 30s or less contact time or 15 s or less contact time, the European standard testing protocol EN13624.

Exemplary compositions of the disclosure can display high levels of antimicrobial efficacy even in "medical dirty conditions" (tests EN13727 and/or EN13624) while also providing pleasing skin feel, pH compatibility with skin and without causing dermal or eye irritation.

In addition to the aforementioned tests and testing protocols, the skin disinfectant compositions or formulations prepared according to the present disclosure may pass the stringent European standard washing norm EN 1499 with a 60 s exposure time. Certain formulations may pass the European standard washing norm EN 1499 with a 30 s exposure time.

European standard testing protocol EN1499 is a rigorous test that uses a cohort of volunteers to simulate real-world hand washing conditions. As part of the test, the volunteers' hands are artificially contaminated, and the volunteers split into a control group receiving a standard soap and a test group receiving the skin disinfectant composition. By comparing the two groups, a time for reaching a statistically significant reduction in bacteria can be determined.

Both European standard testing protocol EN1499 as well as the relevant bactericidal and yeasticidal suspension tests must be passed with the composition demonstrating the required efficacy in order for said composition to be registered as an antimicrobial skin disinfectant composition.

**Disinfectant**

A disinfectant is a product or composition that is capable of chemical disinfection to produce a state in which the number of living/viable microorganisms has been reduced to a level which is judges to be appropriate for a defined purpose or practical situation, e.g. a level low enough to limit the release of microorganisms in numbers which could cause transmission of infection or disease by the irreversible action of a product on their structure or metabolism. An antimicrobial skin disinfectant composition may be described as a formulation or product with a demonstrated biocidal activity when tested against a limited range of microbial species chosen as representative species taking into account their relative resistance and their relevance to practical use in the context of skin disinfection. For a product or composition having skin disinfectant properties suitable for use in medical, food, industrial, domestic and institutional areas, the product shall be tested in accordance with and shall conform to the relevant test standards, such as EN13727, EN13624 and similar or equivalent testing protocols.

**Skin and Eye Irritation according to the Classification, Labelling and Packaging (CLP)**

In addition to anti-bacterial, fungicidal or yeasticidal efficacy the disinfectant compositions formulated according to the disclosure may not cause any skin irritation according to the CLP (Classification, Labelling and Packaging) principles used for hazard classification assessment following the principles outlined in CLP 1272/2008 and the supporting guidance document 'Guidance on the Application of the CLP Criteria' designed to provide the CLP definitions of the hazard endpoints deemed to be significant in relation to a Hand Disinfectant Soap; specifically skin corrosion/irritation and serious eye damage/eye irritation.

Skin irritation means the production of reversible damage to the skin following the application of a test substance for up to 4 hours.

Skin corrosion means the production of irreversible damage to the skin; namely, visible necrosis through the epidermis and into the dermis, following the application of a test substance for up to 4 hours.

In addition to anti-bacterial, fungicidal or yeasticidal efficacy the disinfectant compositions formulated according to the disclosure may not cause any serious eye irritation according to the CLP (Classification, Labelling and Packaging) principles used for hazard classification assessment following the principles outlined in CLP 1272/2008 and the supporting guidance e document 'Guidance on the Application of the CLP Criteria' designed to provide the CLP definitions of the hazard endpoints deemed to be significant in relation to a Hand Disinfectant Soap; specifically skin corrosion/irritation and serious eye damage/eye irritation.

Serious eye damage means the production of tissue damage in the eye, or serious physical decay of vision, following application of a test substance to the anterior surface of the eye, which is not fully reversible within 21 days of application.

Eye irritation means the production of changes in the eye following the application of test substance to the anterior surface of the eye, which are fully reversible within 21 days of application.

In addition to anti-bacterial, fungicidal or yeasticidal efficacy the disinfectant compositions formulated according to the disclosure may not cause any serious eye irritation and may not cause any serious skin irritation according to the CLP (Classification, Labelling and Packaging) principles used for hazard classification assessment following the principles outlined in CLP 1272/2008 and the supporting guidance document 'Guidance on the Application of the CLP Criteria' designed to provide the CLP definitions of the hazard endpoints deemed to be significant in relation to a Hand Disinfectant Soap; specifically skin corrosion/irritation and serious eye damage/eye irritation.

Example disinfectant compositions formulated according to the disclosure may not be classified as hazardous under CLP according to the CLP (Classification, Labelling and Packaging) principles used for hazard classification assessment following the principles outlined in CLP 1272/2008 and the supporting guidance document 'Guidance on the Application of the CLP Criteria' designed to provide the CLP definitions of the hazard endpoints deemed to be significant in relation to a Hand Disinfectant Soap; specifically skin corrosion/irritation and serious eye damage/eye irritation.

**Quaternary ammonium compounds**

The present disclosure provides for a disinfectant compositions comprising one or more quaternary ammonium compounds having biocidal activity, also known as "quats". The one or more quaternary ammonium compounds may operate as a microbial control agent, eliminating and/or destroying certain pathogenic organisms.

Quaternary ammonium compounds typically comprise at least one quaternary ammonium cation with an appropriate anion. Quats will generally have the general formula $R1R2R3R4N^+$, $A^-$; which is equivalent to the general formula (1):

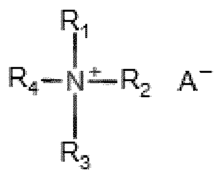

(1)

The groups R1, R2, R3 and R4 can vary within wide limits. Typically, R1, R2 and R3 are independently alkyl groups comprising from 1 to 24 carbon atoms and R4 is an alkyl group comprising from 1 to 24 carbon atoms, a substituted or unsubstituted benzyl group, or an alkoxy group according to the formula: $-[(CH_2)_2\text{-}O]_nR_5$ where n=1-20 and $R_5$ is hydrogen or an unsubstituted or substituted phenyl.

R1, R2 and R3 may be a lower alkyl, meaning having 1 to 4 carbon atoms, such as a methyl, ethyl, propyl or butyl group and R4 may be an alkyl comprising from 6 to 24 carbon atoms, a substituted or unsubstituted benzyl group, or an alkoxy group according to the formula: $-[(CH_2)_2\text{-}O]_nR_5$ where n=1-20 and $R_5$ is hydrogen or an unsubstituted or substituted phenyl. Likewise, R1 and R2 may be a lower alkyl, meaning having 1 to 4 carbon atoms, such as a methyl, ethyl, propyl or butyl group, R3 may be an alkyl comprising from 6 to 24 carbon atoms and R4 may be a an alkyl comprising from 6 to 24 carbon atoms or a substituted or unsubstituted benzyl group, or an alkoxy group according to the formula: $-[(CH_2)_2\text{-}O]_nR_5$ where n=1-20 and $R_5$ is hydrogen or an unsubstituted or substituted phenyl.

$A^-$ may be a monovalent anion or one equivalent of a polyvalent anion of an inorganic or organic acid. Suitable

anions for A are in principle all inorganic or organic anions, in particular halides, for example chloride or bromide, carbonates, bicarbonates, carboxylates, sulfonates, phosphates, propionates, saccharinates, sulphates, ethyl sulphates or a mixture thereof. Carboxylates may be derived from lower carboxylic acids or from fatty acids.

Alkyl, hereinafter, is taken to mean in each case unbranched or branched alkyl groups of the specified number of carbons, but preferably unbranched alkyl groups, and particularly preferably those having an even number of carbon atoms. In particular, this is also taken to mean the homologue mixtures derived from natural raw materials, for example "cocoalkyl".

Disclosed are quaternary ammonium compounds that have the following R groups: R1, R2 and R3 are alkyl groups and R4 is a substituted or unsubstituted benzyl group, a C1-18 alkyl group such as a C6-18 alkyl group, or an alkoxy group such as a group having the structure $-[(CH_2)_{2-}O]_nR_5$ where n=1-20 and $R_5$ is hydrogen or an unsubstituted or substituted phenyl, and A- is as described above, such as a monovalent anion or one equivalent of a polyvalent anion of an inorganic or organic acid. For example, R1 is an alkyl group having 1 to 4 carbon atoms and R2 and R3 are independently alkyl groups having 6 to 24 carbon atoms, or R1 and R2 are independently alkyl groups having 1 to 4 carbon atoms and R3 is an alkyl group having 6 to 24 carbon atoms.

Substituted phenyl is taken to mean, in particular, phenyl groups substituted with one or more C1-18 alkyl groups and/or halogen atoms.

Substituted benzyl is taken to mean, in particular, benzyl groups substituted with one or more C1-18 alkyl groups and/or halogen atoms.

Suitable quaternary ammonium compounds include, but are not limited to, alkyldimethylbenzyl ammonium chlorides, wherein the alkyl group contains from 1 to 24 carbon atoms; dialkylmethylbenzyl ammonium chlorides, wherein the alkyl group contains from 1 to 24 carbon atoms; dialkyldimethyl ammonium chlorides, wherein the alkyl group contains from 1 to 24 carbon atoms; alkyl dimethyl ethylbenzyl quaternary ammonium chlorides, wherein the alkyl group contains from 1 to 24 carbon atoms; benzethonium chloride and any combination of any of the foregoing. One example on a non-halide or a non-chloride quaternary compound is didecylmethylpoly(oxyethyl) ammonium propionate. Didecylmethylpoy(oxethyl ammonium propionate may be used alone or in combination with any of the quaternary ammonium compounds described above.

A quaternary ammonium compound may generally comprise a dialkyl ammonium compound. According to the present invention, the composition of the invention comprises as quaternary ammonium compound a dimethyl dialkyl ammonium compound, wherein the alkyl group contains from 8 and 12 carbon atoms, such as from 8 to 10 carbon atoms in each of the alkyl groups Examples of dimethyl dialkyl ammonium compounds include dimethyl dioctyl ammonium compounds such as dimethyl dioctyl ammonium chloride, dimethyl didecyl ammonium compounds such as dimethyl didecyl ammonium chloride and the like. Mixtures of dimethyl dialkyl ammonium compounds may also be used, and other anions, such as those described above, may also be used. Commercially available dimethyl dialkyl ammonium compounds include, for example, compositions marketed and sold under the BARDAC®, BARDAP®, BARQUAT®, or CARBOQUAT® trade names by Lonza Inc.

Such commercially available examples of dimethyl dialkyl ammonium compounds include dioctyldimethylammonium chloride (available as Bardac® LF and LF-80 from Lonza, Inc.), octyldecyldimethylammonium chloride (available as a mixture of octyldecyldimethylammonium chloride, dioctyldimethylammonium chloride, and didecyldimethyl ammonium chloride as Bardac® 2050 and 2080 from Lonza, Inc.), didecyldimethylammonium chloride (available as Bardac® 2240, 2270 and 2280 from Lonza, Inc.), decylisononyldimethylammonium chloride (available as Bardac® 21 from Lonza, Inc.), diisodecyldimethylammonium chloride (available as BTC 99 from Stepan Co. of Northfield, Ill.), and any combination of any of the foregoing. In certain embodiments, the quaternary ammonium compound is didecyldimethylammonium chloride.

Disclosed are quaternary ammonium compounds that comprise a benzyl ammonium compound, such as an alkyl dimethyl benzyl ammonium compound. In general, the alkyl group may contain from about 1 to about 24 carbon atoms, such as from about 10 to about 18 carbon atoms, or from about 12 to about 16 carbon atoms.

Examples of alkyl dimethyl benzyl ammonium compounds useable as biocide include C12 alkyl dimethyl benzyl ammonium chloride, C14 alkyl dimethyl benzyl ammonium chloride, and C16 alkyl dimethyl benzyl ammonium chloride. In addition, a mixture of these alkyl dimethyl benzyl ammonium compounds can be used. Commercially available alkyl dimethyl benzyl ammonium compounds include, for example, compositions marketed and sold under the BARQUAT® trade name by Lonza Inc. These commercially available alkyl dimethyl benzyl ammonium compounds are blends of C12, C14, and C16 alkyl dimethyl benzyl ammonium chlorides. Generally, it is preferable that the alkyl dimethyl benzyl ammonium compound, when a blend, contains higher concentrations of C12 alkyl and C14 alkyl components than C16 alkyl components. It is noted that other anions, including those mentioned above may also be used.

Non-limiting examples of alkyldimethylbenzyl ammonium chlorides include alkyl (C14 50%; C12 40%, C16 10%) dimethylbenzyl ammonium chloride (available as Barquat® MB-50 and MB-80 from Lonza Inc.), alkyl (C14 60%; C16 30%; C12 5%. C18 5%) dimethylbenzyl ammonium chloride (available as Barquat® 4280Z from Lonza Inc.), (C12-C18 alkyl) dimethylbenzyl ammonium chloride, and any combination of any of the foregoing.

Disclosed are quaternary ammonium compounds that comprise a quaternary ammonium carbonate. A quaternary ammonium carbonate can be represented by the following formula (R1 $R2R3R4N^+)_2$, $CO_3^{2-}$
wherein R1 is a C1-C24 alkyl or aryl-substituted alkyl group, R2 is a C1-C24 alkyl group, and preferably wherein R1 is the same as R2 and R1 is a C8-C12 alkyl group, and wherein R3 and R4 are independently a C1-C24 alkyl.

Disclosed are quaternary ammonium compounds that comprise a quaternary ammonium bicarbonate of general formula R1 $R2R3R4N^+$, $HCO_3^-$
wherein R1 is the same or a different C1-C24 alkyl or aryl-substituted alkyl group as above and R2 is the same or a different C1-C24 alkyl group as above, but preferably wherein R1 is the same as R2 and R1 is a C8-C12 alkyl group, and wherein R3 and R4 are independently a C1-C20 alkyl. Disclosed are ammonium quaternary compounds that comprise a di C8-C12 alkyl ammonium carbonate/bicarbonate. For example, the quaternary ammonium compound may comprise didecyl dimethyl ammonium carbonate and didecyl dimethyl ammonium bicarbonate.

The carbonate/bicarbonate salts of quaternary ammonium cations may be selected from dioctyldimethylammonium carbonate, decyloctyldimethylammonium carbonate, benzalkonium carbonate, benzethonium carbonate, stearalkonium carbonate, cetrimonium carbonate, behentrimonium carbonate, dioctyldimethylammonium bicarbonate, decyloctyldimethylammonium bicarbonate, benzalkonium bicarbonate, benzethonium bicarbonate, stearalkonium bicarbonate, cetrimonium bicarbonate, behentrimonium bicarbonate, and mixtures of one or more such carbonate salts.

Disclosed are quaternary ammonium compounds that comprise a quaternary ammonium sulphate or ethyl sulphate.

Non-limiting examples of sulphate or ethyl sulphate quaternary ammonium compounds include mecetronium ethyl sulphate.

It should be understood that any combination of the quaternary ammonium cations R1 R2R3R4N+ and anions A- aforementioned may be possible. It should be understood that the quaternary ammonium compound may comprise more than one specific quaternary ammonium species and may comprise a combination of any of the above described quaternary ammonium compounds. The one or more quaternary ammonium compounds comprise a dimethyl dialkyl ammonium chloride wherein each alkyl group contains 8 to 12 carbon atoms.

Preferably, the quaternary ammonium compound is selected from the group consisting of didecyldimethylammonium chloride (DDAC). The skin disinfectant composition may further comprise benzalkonium chloride.

Generally, the concentration of the quaternary ammonium compound should be within an active range having a lower value and an upper value. The quaternary ammonium compound(s) is present in the disinfectant composition in an amount of from 0.1 wt% to 2.0 wt% based upon the total weight of the composition. In certain embodiments, the quaternary ammonium compound(s) may be present in the disinfectant composition in an amount of from 0.2 wt% to 1.0 wt%, based upon the total weight of the composition. In certain embodiments, the quaternary ammonium compound(s) may be present in the disinfectant composition in an amount of from 0.1 wt% to 0.7 wt%, such as from 0.1 wt% to 0.6 wt%, based upon the total weight of the composition. In certain embodiments, the quaternary ammonium compound(s) may be present in the disinfectant composition in an amount of from 0.2 wt% to 0.6 wt%, based upon the total weight of the composition.

**Surfactant**

In combination with the one or more quaternary ammonium compounds, one or more surfactants may be present. The surfactant(s) are selected from the group consisting of non-ionic surfactants, amphoteric surfactants and mixtures thereof.

In certain compositions, formulated in accordance with the disclosure, the one or more surfactants may comprise at least a non-ionic surfactant.

Some exemplary, but not limiting, non-ionic surfactants that can be included in formulations of the present disclosure include ethoxylated fatty alcohols; block copolymers of polyethylene glycol and polypropylene glycol, also called polyoxyethylene-polyoxypropylene block copolymers, such as poloxamers; alkyl glucosides, such as decyl glucoside, sodium lauryl glucose carboxylate, lauryl glucoside and capryl glucoside; glycerol alkyl esters such as glyceryl laurate; polyglyceryl esters; alkyl glucosides or polyglucosides such as C8, C10, C12, C14, C16 polyglucosides or any combination thereof; glucoside alkyl ethers; polyoxyethylene glycol alkyl ethers or polyethylene glycol alkyl ethers, such as polyethylene glycol (23) lauryl ether, C9-11 hexaethylene glycol alkyl ethers, polyoxyethylene (21) stearyl ether, polyoxyethylene (4) lauryl ether, polyoxyethylene (10) cetyl ether, polyoxyethylene (20) cetyl ether, octaethylene glycol monododecyl ether, and pentaethylene glycol monododecyl ether; polyoxypropylene glycol alkyl ethers; polyoxyethylene glycol octylphenol ethers; polyoxyethylene glycol alkylphenol ethers, such as nonoxynol-9; sorbitan alkyl esters; polyoxyethylene glycol sorbitan alkyl esters and fatty acid esters such as polysorbate 20 (also called polyoxyethylene (20) sorbitan monolaurate), polysorbate 40 (also called polyoxyethylene (20) sorbitan monopalmitate, polysorbate 60 (also called polyoxyethylene (20) sorbitan monostearate), polysorbate 80 (also called polyoxyethylene (20) sorbitan monooleate); polyoxyethylene fatty acid esters such as polyoxyl 40 stearate, polyoxyl 40 oleate, polyoxyl 8 stearate, polyoxyl

15 hydroxystearate or any combination thereof; polyethyleneglycol sorbitan fatty acid esters such as PEG-80 sorbitan esters; fatty acid amides and their ethoxylates such as cocamide MEA, cocamide DEA; polyethoxylated tallow amine; mixtures of polyoxyethylene mono- and di-esters of C8-C22 fatty acids and glyceryl mono-, di-, and tri-esters of C8-C22 fatty acids such as caprylocaproyl macrogol-8 glycerides, oleoyl macrogol-6 glycerides or linoleoyl macrogol-6 glycerides, lauroyl macrogol-32 glycerides, stearoyl macrogol-32 glycerides and macrogol stearate; polyethoxylated castor oils and derivatives such as polyoxyl 35 castor oil; polyoxyl 40 hydrogenated castor oil, polyoxyl 60 hydrogenated castor oil or any combination thereof; alkanolamines; polyoxythylene silicones; N-alkylpyrrolidones and any combinations of any of the foregoing.

Polyglyceryl esters useable in the present disclosure may be formed from saturated, unsaturated, natural or synthetic fatty acids, and the like. For instance, saturated fatty acids include caprylic acid, capric acid, lauric acid, myristic acid, palmitic acid, stearic acid, arachidic acid, behenic acid, lignoceric acid, cerotic acid, combinations thereof, derivatives thereof, and the like. Furthermore, the polyglyceryl esters are derived from (a) a polyglycerol component built up from 2 to 12 molecules of glycerol, based on an average, and (b) a fatty acid selected from the group consisting of caprylic acid, capric acid, lauric acid, myristic acid, palmitic acid, stearic acid, arachidic acid, behenic acid, lignoceric acid, cerotic acid, oleic acid, decaoleic acid, mixtures thereof and the like.

Examples of polyglyceryl esters useable in the present disclosure, include but are not limited to, polyglyceryl monodecaoleate such as polyglyceryl-10 decaoleate; polyglyceryl monooleate such as polyglyceryl-2-monooleate, polyglyceryl-3 monooleate, polyglyceryl-4 monooleate, polyglyceryl-6 monooleate, or polyglyceryl-10 monooleate; polyglyceryl dioleate such as polyglyceryl-2 dioleate, polyglyceryl-3 dioleate, polyglyceryl-5 dioleate, polyglyceryl-6 dioleate or polyglyceryl-10 dioleate; polyglyceryl trioleate such as polyglyceryl-5 trioleate or polyglyceryl-10 trioleate; polyglyceryl tetraoleate such as polyglyceryl-2 tetraoleate, polyglyceryl-6 tetraoleate, or polyglyceryl-10 tetraoleate; polyglyceryl pentaoleate such as polyglyceryl-4 pentaoleate, polyglyceryl-6 pentaoleate, or polyglyceryl-10 pentaoleate; polyglyceryl heptaoleate such as polyglyceryl-6 heptaoleate, polyglyceryl-10 heptaoleate; polyglyceryl monostearate such as polyglyceryl-2 monostearate, polyglyceryl-3 monostearate, polyglyceryl-4 monostearate, polyglyceryl-5 monostearate, polyglyceryl-6 monostearate or polyglyceryl-10 monostearate; polyglyceryl distearate such as polyglyceryl-2 distearate, polyglyceryl-3 distearate, polyglyceryl-4 distearate, polyglyceryl-6 distearate, or polyglyceryl-10 distearate; polyglyceryl tristearate such as polyglyceryl-4 tristearate, polyglyceryl-5 tristearate, polyglyceryl-6 tristearate, or polyglyceryl-10 tristearate; polyglyceryl tetrastea rate such as polyglyceryl-2 tetrastearate; polyglyceryl pentastearate such as polyglyceryl-4 pentastearate, polyglyceryl-6 pentastearate, or polyglyceryl-10 pentastearate; polyglyceryl heptastearate such as polyglyceryl-10 heptastearate; polyglyceryl isostearate such as polyglyceryl-2 isostearate, polyglyceryl-3 isostearate, polyglyceryl-4 isostearate, polyglyceryl-6 isostearate, or polyglyceryl-10 isostearate; polyglyceryl diisostearate such as polyglyceryl-2 diisostearate. polyglyceryl-3 diisostearate, polyglyceryl-4 diisostearate, polyglyceryl-6 diisostearate, polyglyceryl-10 diisostearate, or polyglyceryl-15 diisostearate; polyglyceryl triisostearate such as polyglyceryl-2 triisostearate, polyglyceryl-3 triisostearate, polyglyceryl-5 triisostearate, polyglyceryl-10 triisostearate; polyglyceryl tetraisostearate such as polyglyceryl-2 tetraisostearate; polyglyceryl caprylate such as polyglyceryl-2 caprylate, polyglyceryl-3 caprylate, polyglyceryl-4 caprylate, polyglyceryl-6 caprylate, or polyglyceryl-10 caprylate; polyglyceryl dicaprylate such as polyglyceryl-5 dicaprylate; polyglyceryl sesquicaprylate such as polyglyceryl-2 sesquicapyrlate; polyglyceryl octacaprylate such as polyglyceryl-6 octacaprylate; polyglyceryl caprate such as polyglyceryl-2 caprate, polyglyceryl-3 caprate, polyglyceryl-4 caprate, polyglyceryl-5 caprate, polyglyceryl-6 caprate, polyglyceryl-10 caprate, polyglyceryl dicaprate such as polyglyceryl-3 dicaprate or polyglyceryl-6 dicaprate; polyglyceryl caprylate/caprate such as polyglyceryl-4 capyrlateicaprate, polyglyceryl-6 caprylate/caprate, or polyglyceryl-10 caprylate/caprate; polyglyceryl palmitate such as polyglyceryl-2 palmitate, polyglyceryl-3 palmitate, polyglyceryl-6 palmitate or polyglyceryl-10 palmitate; polyglyceryl dipalmitate such as polyglyceryl-6 dipalmitate or polyglyceryl-10 dipalmitate; polyglyceryl tetrabehenate such as polyglyceryl-6 tetrabehenate; polyglyceryl myristate such as polyglyceryl-6 myristate or polyglyceryl-10 myristate; polyglyceryl rincinoleate such polyglyceryl-6 polyricinoleate or polyglyceryl-10 ricinoleate; or mixtures thereof, other complexes or derivatives thereof, and the like.

Suitably, the polyglyceryl ester may be one or more of a polyglyceryl-10 decaoleate, polyglyceryl-3 monostearate, polyglyceryl-6 distearate, polyglyceryl-10 stearate, polyglyceryl-10 oleate, polyglyceryl-10 dipalmitate, polyglyceryl-10 caprylate/caprate; and a mixture thereof. In one aspect, the polyglyceryl ester is polyglyceryl-10 caprylate/caprate.

In one embodiment, the non-ionic surfactant is selected from the group consisting of polyglyceryl-10 caprylate/caprate, polysorbate 20, polysorbate 80, decyl glucoside, polyethylene glycol (23) lauryl ether, C9-11 hexaethylene glycol alkyl ethers and polyoxyethylene (21) stearyl ether.

In certain compositions, formulated in accordance with the disclosure, the one or more surfactants may comprise at least an amphoteric surfactant.

Several non-limiting examples of amphoteric surfactants than can be included in compositions of the disclosure include beta-n-alkylaminopropionic acids, n-alkyl-beta-iminodipropionic acids, imidazoline carboxylates, amine oxides, sultaines, betaines, phosphocholines, propionates, di-propionates, sodium cocoamphoacetate, disodium cocoamphodiacetate, 3-[(3-Cholamidopropyl)dimethylammonio]-1-propanesulfonate (CHAPS), dipalmitoylphosphatidylcholine

(DPPtdCho), sodium lauroamphocetate and any combinations of any of the foregoing.

Several non-limiting examples of amine oxides include octyl dimethyl amine oxide (i.e. Barlox 8S), decyl dimethyl amine oxide (i.e. Barlox 10S), lauryldimethylamine oxide (i.e. Barlox ® 12), cocoalkyldimethyl amine oxide, myristyldimethyl amine oxide (i.e. Barlox ® 14), octyldimethyl amine oxide, decyldimethyl amine oxide, lauryldimethyl amine oxide, isoalkyl dimethyl amine oxide tetradecyldimethylaminoxide and cetyldimethyl amine oxide. Several non-limiting examples of betaines include lauramidopropyl betaine, cocamidopropyl betaine (i.e. and Dehyton® PK45), lauramidopropyl betaine, cetyl betaine, oleamidopropyl betaine and ricinoleamidopropyl betain. Several non-limiting examples of sultaines include cocamidopropyl hydroxysultaine, lauramidopropyl hydroxysultaine, cocamidopropyl hydroxysultaine, oleamidopropyl hydroxysultaine, tallowamidopropyl hydroxysultaine, erucamidopropyl hydroxysultaine and lauryl hydroxysultaine. Several non-limiting examples of phosphocholines include dodecylphosphocholine. Several non-limiting examples of propionates include ß-Alanine, N-(2-carboxyethyl)-, N-coco alkyl derivatives. Several non-limiting examples of di-propionates include disodium capryloamphodipropionate and disodium cocoamphodipropionate.

In one embodiment, the amphoteric surfactant is selected from the group consisting of octyldimethyl amine oxide, decyldimethyl amine oxide, lauryldimethyl amine oxide, isoalkyl dimethyl amine oxide, tetradecyldimethyl amine oxide, cetyldimethyl amine oxide, cocoamidopropyl betaine and ß-Alanine, N-(2-carboxyethyl)-, N-coco alkyl derivatives.

In certain compositions, the one or more surfactants may comprise at least one amphoteric surfactant and at least one non-ionic surfactant.

Preferably, the one or more surfactants are selected from the group consisting of polysorbate 20, lauryl dimethyl amine oxide, polyglyceryl-10 Caprylate/Caprate, and combinations thereof.

The compositions of the present invention do not contain an anionic surfactant.

The total concentration of the one or more surfactant(s) (either one or more non-ionic, one or more amphoteric or mixtures thereof) is from 0.1 wt%, 0.3 wt% or 1.0 wt%, based upon the total weight of the composition to an upper value of 3.0 wt% or 4.0 wt%, based upon the total weight of the composition. Compositions formulated according to the disclosure can have a concentration of the one or more surfactants based upon the total weight of the composition within the range of any one lower bound to any upper bound inclusive the end values. Several non-limiting examples of ranges for the concentration of the one or more surfactants in accordance with the disclosure can include: 0.1 wt% to 3.0 wt%, about 0.3 wt% to 3.0 wt%,0.1 wt% to 4.0 wt%, and 1.0 wt% to 4.0 wt% based upon the total weight of the composition.

In certain compositions of the present disclosure comprising a mixture of at least non-ionic surfactant and at least an amphoteric surfactant, the amphoteric surfactant may be an amine oxide present at a concentration from 0.1 wt% to 3.0 wt% or at a concentration from about 0.1 to 1.0 wt%, or at a concentration from 0.1 to 0.5 wt%, based upon the total weight of the composition.

**Aromatic Alcohol**

In combination with the quaternary ammonium compound and the one or more surfactants, an aromatic alcohol is be present, wherein the aromatic alcohol is phenoxyethanol. In general, an aromatic alcohol is defined in the present application as a compound having a structure that includes at least one aromatic group (e.g., phenyl, benzyl, naphthyl, etc.) substituted with a carbon chain including an alcohol group. Several non-limiting examples of aromatic alcohols are disclosure: 2-phenoxyethanol, benzyl alcohol, phenoxyisopropanol, chlorocresol, 2-phenylphenol, and 5-chloro-2-(4-chlorphenoxy)phenol. In certain embodiments, the aromatic alcohol is 2-phenoxyethanol. The concentration of the aromatic alcohol in accordance with the invention is from 0.1 wt% to 1.0 wt%, based upon the total weight of the composition

In the compositions formulated according to the invention the aromatic alcohol is present in an amount of from 0.1 to 1.0 wt% based upon the total weight of the composition and the one or more surfactants comprises at least one amphoteric surfactant present in an amount of from 0.1 to 4.0 wt%, based upon the total weight of the composition.

In certain compositions, the aromatic alcohol is present in an amount of from 0.1 to 1.0 wt% based upon the total weight of the composition and the one or more surfactants comprises at least one amine oxide present in an amount of from 0.1 to 4.0 wt%, based upon the total weight of the composition.

In other compositions, the aromatic alcohol may be present in an amount of from 0.1 to 1.0 wt% based upon the total weight of the composition and the one or more surfactants may comprise at least one amine oxide present in an amount of from 0.1 to 3.0 wt%, based upon the total weight of the composition.

In certain compositions the aromatic alcohol is present in an amount of from 0.1 to 0.5 wt% based upon the total weight of the composition, the one or more surfactants comprise at least a non-ionic surfactant and at least an amphoteric surfactant in an amount of from 1.0 to 4.0 wt% based upon the total weight of the composition and the weight ratio of amphoteric surfactant to non-ionic surfactant is 1:1 to 1:6.

In one embodiment, the skin disinfectant composition of the present invention comprises:

a. one or more quaternary ammonium compounds selected from didecyldimethylammonium chloride in an amount of from 0.1 to 0.7 wt.%, based upon the total weight of the composition;

b. one or more surfactants selected from polysorbate 20, lauryl dimethyl amine oxide, and polyglyceryl-10 caprylate/caprate in an amount of from 0.3 to 3.0 wt%, based upon the total weight of the composition; and
c. 2-phenoxyethanol in an amount of from 0.1 to 1.0 wt%, based upon the total weight of the composition.

The composition does not contain an anionic surfactant.
In one embodiment, in the skin disinfectant composition of the present invention, the one or more quaternary ammonium compound is didecyldimethylammonium chloride (DDAC), the one or more surfactant is polysorbate 20, and the aromatic alcohol is phenoxyethanol.
In one embodiment, in the skin disinfectant composition of the present invention, the one or more quaternary ammonium compound is didecyldimethylammonium chloride (DDAC), the one or more surfactants are polysorbate 20 and lauryl dimethyl amine oxide, and the aromatic alcohol is phenoxyethanol.
Disclosed are skin disinfectant compositions, wherein the one or more quaternary ammonium compound is benzalkonium chloride, the one or more surfactant is polyglyceryl-10 caprylate/caprate, and the aromatic alcohol is phenoxyethanol.
In one embodiment, the skin disinfectant composition of the present invention comprises:

a. didecyldimethylammonium chloride (DDAC) in an amount of from 0.1 to 0.7 wt.%, based upon the total weight of the composition;
b. polysorbate 20 in an amount of from 0.3 to 3.0 wt%, based upon the total weight of the composition; and
c. 2-phenoxyethanol in an amount of from 0.1 to 1.0 wt%, based upon the total weight of the composition.

In one embodiment, the skin disinfectant composition of the present invention comprises:

a. didecyldimethylammonium chloride (DDAC) in an amount of from 0.1 to 0.7 wt.%, based upon the total weight of the composition;
b. one or more surfactants selected from polysorbate 20 and lauryl dimethyl amine oxide in an amount of from 0.3 to 3.0 wt%, based upon the total weight of the composition; and
c. 2-phenoxyethanol in an amount of from 0.1 to 1.0 wt%, based upon the total weight of the composition.

## Chelant

Optionally, the composition may comprise one or more chelators. Several non-limiting examples of chelators include methyl glycine diacetic acid or a salt thereof, ethylenediaminetetraacetic acid (EDTA) or a salt thereof, ethylenediamine N, N'-disuccinic acid (EDDS), trisodium salt of methylglycinediacetic acid (Na3MGDA), Glutamic acid, N,N-diacetic acid, tetrasodium salt (GLDA-Na4) and 1-hydroxyethylidenediphosphonic acid (HEDP). In some of the embodiments the chelating agent may be present in an amount from 0 to 5 wt%, in other embodiments from 0.05 to 1 wt%, based upon the total weight of the composition and in further embodiments from 0.05 to 0.2 wt%.

## pH

Example antimicrobial compositions may include a formulation pH. Generally, the formulation pH can include a lower pH and an upper pH that together define a range of formulation pH in accordance with the disclosure. For example, an aspect of the disclosure can include compositions having a lower pH of about 2.5, 2.8, 3.0, 3.2, 3.5, or 3.7. Another aspect of the disclosure can include compositions having an upper pH of about 7.3, 7.5, 7.7, 8.0, 8.3, 8.6, 9.0. Compositions formulated according to the disclosure can have a formulation pH within the range of any one lower pH to any one upper pH inclusive of the end values. Several non-limiting examples of ranges for the formulation pH in accordance with the disclosure can include: about 2.5 to about 9.0, about 3.2 to about 8.0, and about 3.7 to about 7.0.
Maintaining pH stability can be important for certain compositions formulated in accordance with the disclosure. For certain formulations, the composition may further include one or more buffers for maintaining the formulation pH. Several non-limiting examples of solution buffers can include: glycine-HCl, acetic acid, citric acid, lactic acid, glycolic acid, malic acid, succinic acid and tartaric acid, as well as the conjugates bases for these acids. Typically less than 1% by weight of these acids are used to achieve the proper pH.
For certain formulations the pH may be checked or adjusted with hydrochloric acid or sodium hydroxide.

## Humectant

In certain compositions, formulated in accordance with the disclosure, a humectant or skin conditioning agent may also be included in the composition. Several non-limiting examples of humectants than can be included in compositions of the disclosure include amino acids, pyrrolidone carboxylic acid, lactic acid and salts thereof, lactitol, urea and urea derivatives, uric acid, glucosamine, creatinine, cleavage products of collagen, chitosan or chitosan salts/derivatives and, in particular, polyols and polyol derivatives, for example glycerol, diglycerol, triglycerol, polyglycerin, such as polyglycerin-6, ethylene glycol, propylene glycol, dipropylene glycol, butylene glycol, erythritol, 1,2,6-hexanetriol, polyethylene glycols, sugars and sugar derivatives (e.g. fructose, glucose, maltose, maltitol, mannitol, inositol, sorbitol, sorbitol silanediol, sucrose, trehalose, xylose, xylitol, glucuronic acid and salts thereof), ethoxylated sorbitol (Sorbeth-6, Sorbeth-20, Sorbeth-30, Sorbeth-40), honey and hydrogenated honey, hydrogenated starch hydrolyzates and mixtures of hydrogenated wheat protein and PEG-20-acetate copolymer, and the like.

In an example, the humectant can be present at a concentration of about 0% to about 7% by weight. In another example, the humectant can be present at a concentration of about % to about 6% by weight. In a further example, the humectant can be present at a concentration of about 4.2% to about 5.5% by weight, based upon the total weight of the composition.

### $H_2O_2$ or other oxidizing agents

In an example formulation, an oxidizing agent may be included. Non-limiting examples or oxidizing agents can include hydrogen peroxide or a precursor or adduct that forms hydrogen peroxide. The hydrogen peroxide sources include, but are not limited to hydrogen peroxide solutions, percarbonate salts (i.e. sodium percarbonate, potassium percarbonate), perborate salts (i.e. sodium perborate, potassium perborate), hydrogen peroxide urea or peroxide salts, hydrated salts thereof, or combinations thereof, and the like. Generally, the concentration of the oxidizing agent should be within an active range having a lower bound and an upper bound. An aspect of the disclosure can include disinfectant compositions having a lower bound of about 0.8%, 1.0%, 1.2%, or 1.5%.

Another aspect of the disclosure can include disinfectant conditions having an upper bound of about 2.5%, 2.8%, 3.0%, or 3.5%. Disinfectant compositions formulated according to the disclosure can have a concentration of the oxidizing agent based on the total weight of the disinfectant composition within the range of any one lower bound to any one upper bound inclusive of the end values. Several non-limiting examples of ranges for the concentration of the oxidizing agent in accordance with the disclosure can include: about 0.8% to about 3.5%, about 1.0% to about 3.0%, and about1.5% to about 2.8%.

In compositions comprising an oxidizing agent, such as hydrogen peroxide, a stabilizer, such as a peroxide stabilizer may be included. Suitably examples, include, but are not limited to, lactic acid, phosphoric acid, 1-hydroxyethylidenediphosphonic acid (HEDP), phytic acid, aminophosphate, phosphonate and sodium glutamate, $NaH_2PO_4$, $Na_5P_3O_{10}$, organophosphonic acid, aminophosphonate, silver dihydrogen citrate, diphosphonic acid, ethylenediaminetetraacetic acid (EDTA), N-(hydroxyethyl)-ethylenediaminetriacetic acid (HEDTA), tri(methylene phosphoric acid), diethylenetriaminepenta(methylene phosphoric acid), 2-hydroxy ethylimino bis(ethylene phosphoric acid), citric acid, dipicolinic acid, ethylenediamine-N,N'-disuccinic acid, methylglycinediacetic acid and their alkaline salts thereof, nitriotriacetic acid (NTA), 2-hydroxyethylimino-diacetic acid (HEIDA), and salts thereof, cyclohexane-1 ,2-diaminotetrakismethylene phosphonic acid or water-sol, diethylenetriamine penta(methylene phosphonic acid), colloidal stannate, diethylenetriamine pentaacetic acid (DTPA), citrate salts, gallate salts, malate salts, malonate salts, oxaloacetate salts, oxalate salts, pyruvate salts, succinate salts, 2-hydroxypyridine-1-oxide (2-HPNO), hyroxyethylidene diphosphonic acid (HEDP) zinc salt, triethanol amine phosphate or mixtures thereof, and the like.

If stabilizers and/or chelating agents are added, the stabilizer includes, but is not limited to, peroxide stabilizers, present in an amount from 0.01 w/w% - 5 w/w% based on the weight of the total composition.

In addition to antimicrobial efficacy, disinfectant compositions formulated according to the disclosure may display chemical stability such that active ingredients (e.g., hydrogen peroxide) do not significantly degrade even at elevated temperatures. For example, certain formulations may display a chemical stability such that less than 10% weight reduction in hydrogen peroxide is detected after 2 weeks at 54 °C. In some formulations, the chemical stability can result in less than 10% weight reduction in hydrogen peroxide after 3 months at 40 °C.

### Polymeric Thickeners

For certain applications, disinfectant compositions formulated according to the disclosure may provide particular advantages by providing a suitably-thickened liquid having a viscosity greater than or equal to about 100 cps, such as greater than or equal to about 150 cps, or greater than or equal to about 200 cps. In these disinfectant compositions, a thickener such as a polymer may be included to modify the viscosity. Several non-limiting examples of thickeners include: PEG-120 methyl glucose trioleate (and) propanediol, hydroxyethylcellulose (HEC) and hydrophobically modified hydroxyethylcellulose, hydroxyethylmethylcellulose (HMC) and hydrophobically modified hydroxyethylmethylcellulose, carboxymethylcellulose sodium salt, polyacrylic acid, polystyrene, polyvinyl alcohol, agar, alginate, carrageenan, gum tragacanth, xanthan gum, collagen, glyceryl monostearate, an esterified ethoxylated methyl glucose ether, polyethylene

glycol (PEG) and polyvinylpyrrolidone. Though not required for disinfectant compositions of the disclosure, suitably-thickened compositions can be conveniently used with most liquid and foaming soap dispensers and can provide users with a pleasing skin feel that readily coats the area to be disinfected without running, thus ensuring more complete disinfection.

### Fragrances

In accordance with the disclosure, some formulations may further include a fragrance such as an essential oil. Generally, a fragrance can be any compound or combinations of compounds that produce an odor that can be detected by a person. For some formulations, the fragrance can be synthetically derived (i.e., produced through chemical synthesis rather than obtained from a living organism.) In other formulations, the fragrance can be naturally derived (e.g., by extraction of the fragrance from a plant.) Several non-limiting examples of fragrances (natural or synthetic) that can be used to formulate disinfectant compositions in accordance with the disclosure include: vanilla, lemon, lavender, cedar, cardamom, cinnamon, sage, rose and rosemary.

### Other additives

In accordance with the disclosure, some formulations may also include a product appearance modifier. Aspects of the product appearance modifier can include adjusting the color, luster, and/or opacity of the disinfectant composition. For example, a disinfectant composition formulated in accordance with the disclosure can include at least one product appearance modifier such as a pearlizer. An example pearlizer may contains one or more fatty esters of ethylene glycol. Additionally or alternatively, the pearlizer can contain one or more coated micas.

The skin disinfectant compositions formulated according to the disclosure may further comprise colouring agents, opacifying agents, vitamins, antioxidants, emollients, skin care additives, solvent(s), polymeric thickeners and foaming agents.

The skin disinfectant compositions formulated according to the disclosure may further comprise humectants, solvents, colouring agents or dyes, opacifying agents, pearlizing agents, vitamins, antioxidants, emollients, skin care additives, polymeric thickeners foaming agents, or mixtures thereof.

The balance of the composition is typically water or other non-alcoholic solvent so as to provide 100 percent by weight of the composition. Preferably, no alcohol solvent is used; although, if alcohol were used, it would not materially affect the nature of the composition other than in ways previously disclosed hereinabove.

### Applications

In another aspect, there is provided a hand soap in the form of a liquid, gel or foam comprising, consisting essentially or consisting of the skin disinfectant composition of the present disclosure. The present disclosure may be directed to disinfectant compositions, such as hand soaps or rinse-off hand soaps that can be applied to a portion of the body as well as concentrates of these disinfectant compositions. The disinfectant compositions of the present disclosure can be in the form of a liquid, gel or foam.

The disinfectant compositions of the present invention can be pumped using a hand soap dispenser to provide the disinfectant composition in the form of a liquid, gel, or foam.

The composition according to the disclosure may be applied to a hard or soft surface, a human or animal skin, mucous membrane or air to eliminate microorganisms. A cleansing composition according to the disclosure may be applied in a similar fashion to additional areas of the body or other surfaces.

The composition of the present disclosure can be used for disinfection of substrates, in particular skin or mucous membranes, and / or for disinfection thereof, preferably for disinfection and / or sanitization of hands, face, intimate areas, hair and arms. Next to being suitable for use on the skin the composition may also be used for other disinfecting purposes such as for use on hard surfaces. In another aspect the disclosure relates to a method to reduce the bacteria or yeast population on a surface e.g., a human or animal skin or mucus membrane by contacting the substrate for at least 60 s or for at least 30 s with the composition according to the present disclosure.

It will be clear to the skilled person that the various embodiments and preferences described herein can be combined, unless they are presented as mutually excluding alternatives.

Additionally or alternatively, a disinfectant composition formulated in accordance with the disclosure can be essentially free of a short chain alcohol (e.g., a C1-C5 mono-alcohol or diol.) As used herein a short chain alcohol is defined as an organic compound including an alcohol (C-OH) moiety that has a boiling point of less than about 140 °C at standard temperature and pressure. Example short chain alcohols include: methanol, ethanol, propanol, isopropanol, butanol, pentanol, and isoamyl alcohol. While these alcohols can be used as disinfectant agents due to their dehydrating properties, their mechanism of action can negatively impact skin leading to dryness or redness. Additionally, short chain

alcohols can disrupt surfactants to diminish foaming, which may cause users of disinfection products to be unsure if the product is working or is effective. In certain formulations, disinfection can be effectively achieved using compositions that are essentially free of short chain alcohols and without the drawbacks. Thus short chain alcohols may be excluded in some formulations disclosed herein.

A further benefit of disinfectant compositions of the disclosure includes the physical and chemical stability of the compositions. Generally, separation of a disinfectant formulation is undesirable since it may signal an issue to users or may result in delivery of only some of the disinfection mixture. Thus separation can lead to incomplete disinfection and/or skin irritation due to the incomplete delivery of the composition. An aspect of disinfectant compositions formulated according to the disclosure can also include physically stable compositions, such that the compositions do not phase separate into two layers at a storage temperature.

## EXAMPLES

The skin disinfectant compositions of the disclosure are generally prepared by dissolving the various ingredients quaternary ammonium compounds, aromatic alcohols, the nonionic surfactant(s), and/or the amphoteric surfactant(s) in water. More particularly, suitable surfactant(s), humectant, chelators and the rest of the ingredients if desired (such as viscosity builders, fragrance and pearlizing agent) are dissolved in water with stirring.

The solution is mixed until it is completely hydrated. pH is checked and adjusted if necessary. Any acids compatible with the components of the composition can be used to adjust pH, typically less than 1% by weight of these acids are used to achieve the proper pH. This process can be employed with or without the application of heat to enhance dissolution.

### Classification, Labelling and Packaging (CLP)

CLP outlines the principles for classification of mixtures and approaches mixture classification in a stepwise approach;

1. Using test data on the mixture
2. Using test data for 'similar mixtures' using bridging principles.
3. Taking into consideration the classification of the ingredients of the mixture using the classification thresholds detailed in the regulation

CLP classification was undertaken taking into consideration the classification of the ingredients of the mixture using the classification thresholds detailed in the regulation. For the classification of a skin disinfectant composition, the relevant ingredient principle was disregarded and all components were taken into consideration for classification.

### Additivity approach

Additivity is a principle under CLP where by, if the sum of the concentrations of one or several substances classified for the same hazard class/category in the mixture equals or exceeds the generic concentration limit set out for this hazard class/category, the mixture must be classified for that hazard.

When the supplier is unable to derive the classification using either data on the mixture itself or bridging principles, they must determine the skin corrosion/irritation properties of the mixture using data on the individual ingredients. Although the general approach is the additivity principle, the supplier must ascertain whether the additivity approach is applicable.

For components classified for skin corrosion/irritation and/or serious eye damage/eye irritation the additivity approach generally applies. However, when taking into consideration certain types of substances such as acids and bases, inorganic salts, aldehydes, phenols, and surfactants the additivity approach may not be applicable.

In the case of the skin disinfectant compositions disclosed, the additivity approach was deemed applicable as no components were deemed to fall into a category that would disregard them from the additivity approach.

### Ingredient Classification

In order to classify a mixture based on the classification of the ingredients the first step is to ascertain which hazards are applicable to each ingredient. To do this the following sources were used;

- EU Harmonised Classifications
- Supplier Safety Data Sheets

- ECHA Registration Dossier

Each ingredient in the skin disinfectant compositions evaluated were reviewed and the classification determined on the basis of information found using each applicable source.
Due to new hazard information on substances becoming available the classification will be periodically reviewed and updated as required.
Skin corrosion means the production of irreversible damage to the skin; namely, visible necrosis through the epidermis and into the dermis, following the application of a test substance for up to 4 hours. Corrosive reactions are typified by ulcers, bleeding, bloody scabs, and, by the end of observation at 14 days, by discolouration due to blanching of the skin, complete areas of alopecia, and scars. Histopathology shall be considered to evaluate questionable lesions.
Skin irritation means the production of reversible damage to the skin following the application of a test substance for up to 4 hours.

**Classification Principles**

In general, the approach to classification of mixtures as corrosive or irritant to skin when data are available on the ingredients, but not on the mixture as a whole, is based on the theory of additivity. Each skin corrosive or skin irritant ingredient contributes to the overall skin corrosive or skin irritant properties of the mixture in proportion to its potency and concentration. A weighting factor of 10 is used for skin corrosive ingredients when they are present at a concentration below the generic concentration limit for classification with Category 1, but are at a concentration that will contribute to the classification of the mixture as skin irritant. The mixture is classified as corrosive or irritant to skin when the sum of the concentrations of such ingredients exceeds a concentration limit.
In the absence of any other information, a mixture is considered corrosive to skin (Skin Corrosion Category 1) if it has a pH ≤ 2 or a pH ≥ 11.5.
CLP determines the threshold for classification as Skin Corrosion / Skin Irritation as follows;

Figure 1 CLP Skin Corrosion / Irritation generic concentration thresholds

| Sum of ingredients classified as: | Concentration triggering classification of a mixture as: | |
|---|---|---|
| | Skin corrosion | Skin irritation |
| | Category 1 | Category 2 |
| Skin corrosion subcategory 1A, 1B, 1C | ≥ 5 % | ≥ 1 % but < 5 % |
| Skin irritation (category 2) | | ≥ 10 wt% |
| (10 x skin corrosion subcategory 1A, 1B, 1C or category 1) + skin irritation (category 2) | | ≥ 10 wt% |

As the skin disinfectant compositions described have a pH of between 2 and 10 the compositions as described does not require classification on the basis of extreme pH. Therefore, classification was undertaken based on the sum of classified ingredients. For classification, the relevant ingredient principle was disregarded and all components were taken into consideration for classification.

**Serious eye damage/eye irritation**

**Definitions**

Serious eye damage means the production of tissue damage in the eye, or serious physical decay of vision, following application of a test substance to the anterior surface of the eye, which is not fully reversible within 21 days of application.
Eye irritation means the production of changes in the eye following the application of test substance to the anterior surface of the eye, which are fully reversible within 21 days of application.

### Classification Principles

In general, the approach to classification of mixtures as seriously damaging to the eye/eye irritant when data are available on the ingredients, but not on the mixture as a whole, is based on the theory of additivity. Each skin corrosive or serious eye damaging/eye irritant ingredient contributes to the overall serious eye damage/eye irritation properties of the mixture in proportion to its potency and concentration. A weighting factor of 10 is used for skin corrosive and serious eye damaging ingredients when they are present at a concentration below the generic concentration limit for classification with Category 1, but are at a concentration that will contribute to the classification of the mixture as eye irritant. The mixture is classified as seriously damaging to the eye or eye irritant when the sum of the concentrations of such ingredients exceeds a concentration limit.

In the absence of any other information, a mixture is considered corrosive to skin (Eye Damage Category 1) if it has a pH ≤ 2 or a pH ≥ 11.5.

CLP determines the threshold for classification as serious eye damage/eye irritation as follows; Figure 2 CLP Eye Damage / Irritation generic concentration thresholds

| Sum of ingredients classified as: | Concentration triggering classification of a mixture as: | |
|---|---|---|
| | Skin corrosion | Skin irritation |
| | Category 1 | Category 2 |
| Skin corrosion subcategory 1A, 1B, 1C + serious eye damage (category 1) | ≥ 5 % | ≥ 1 % but < 5 % |
| Eye irritation (category 2) | | ≥ 10 wt% |
| (10 x skin corrosion subcategory 1A, 1B, 1C or category 1) + serious eye damage (category 1) + eye irritation (category 2) | | ≥ 10 wt% |

### Classification Discussion

As the skin disinfectant compositions described have a pH of between 2 and 10 the compositions as described does not require classification on the basis of extreme pH. Therefore, classification was undertaken based on the sum of classified ingredients. For the classification of the skin disinfectant compositions or formulations according to the disclosure, the relevant ingredient principle was disregarded and all components were taken into consideration for classification.

In order to demonstrate practice of the present disclosure, several antimicrobial cleansing compositions were prepared according to the disclosure and were tested to determine their effectiveness against at least one strain of the particular limiting organism, *candida albicans.* This particular strain is on deposit with and is available to the scientific public from the German Type Culture Collection (DSM no 1376). The compositions of the present disclosure may, where indicated below, have also been studied with respect to other strains of *candida albicans* or other organisms, also available to the scientific public from, but not limited to, the DSM or the American type culture collection (ATCC) under various Accession numbers and such as at least one strain of *enterococcus hirae, staphylococcus aureus, pseudomonas aeruginosa and escherichia coli,* available to the scientific public from the ATCC. Details on the organisms tested and their strains can be found below.

The compositions were subject to either a 30 or 60 s kill study of the challenge bacteria and yeast, with a particular focus on the known limiting organisms *pseudomonas aeruginosa* and *candida albicans.* The suspension test carried out in the examples below are based on the European protocols EN13624 and EN13727 to determine the antimicrobial performance of the tested compositions against yeast and bacteria respectively. For EN13727 suspension test 1 mL dirty medical Bovine serum albumin (BSA) (soil comprised of 3 g BSA and 3 mL of sheep blood per 1000 mL sterile distilled water) is added to 1 mL test suspension (containing 10^7 cfu/ml). The two are allowed to interact for 2 min before adding 8 mL of test product, diluted to 50% with 375 ppm hard water. The test temperature was 20°C. After 30 s, 1 mL of the test suspension is added to 9 mL of neutralizer preventing further action of the product. Plating out the neutralized mixture allows enumeration of any remaining viable cells, which can be compared to the initial test suspension for log reduction to be determined. Passing criteria for EN13727 requires a minimum of a 3 log reduction within 60 s in order for a composition to be regarded as bacteriacidal. The organisms tested against this protocol were *E. coli, E. hirae, P. aeruginosa, S. aureus.*

In particular, EN13727 sets out the following passing criteria in section 5.9.2:
The product shall be deemed to have passed the EN13727 Standard (bactericidal activity) if it demonstrates in a valid

test for handrub and handwash products at 20°C under the conditions defined by EN13727 when the test organisms are *E. coli, E. hirae, P. aeruginosa, S. aureus* at least a:

a) 5 log reduction within 60s under clean conditions (hygienic handrub);
b) 5 log reduction within 300s under clean conditions (surgical handrub);
c) 3 log reduction within 60s under dirty conditions (hygienic handwash);
d) 5 log reduction within 300s under dirty conditions (surgical handwash).

Same protocol is applicable for EN13624 except that the organism tested is C. *albicans* and that it starts with a test suspension starting at 10^6 cfu/mL and a 2 log reduction within 60s is required to pass in order for a composition to be regarded as yeaticidal. In particular, EN13624 sets out the following passing criteria in section 5.9.4:
The product shall be deemed to have passed the EN13624 Standard (yeasticidal activity) if it demonstrates in a valid test for handrub and handwash products at 20°C under the conditions defined by EN13624 when the test organism is C. *albicans* at least a:

a) 4 log reduction within 60s under clean conditions (hygienic handrub);
b) 4 log reduction within 300s under clean conditions (surgical handrub);
c) 2 log reduction within 60s under dirty conditions (hygienic handwash);
d) 4 log reduction within 300s under dirty conditions (surgical handwash).

Tests are validated by; A, showing the water and BSA do not have any significant impact on the organism, B, that the neutralizer used has no significant impact on the organism and C that the neutraliser prevents further antimicrobial activity of the product beyond the time allotted (the product must be neutralized within 10 s to be valid for a 30 s contact time) (EN1276 is same but in dirty conditions with 3.0 g/l Bovine Albumin).

The European standard test protocols mentioned in the present disclosure may be reviewed and updated periodically. This disclosure refers to the European standard test protocols of year 2019 for EN1276, year 2013 for EN13624, year 2019 for EN1650, year 2015 for EN13727 and year 2013 for EN1499.

| Type of Activity | Limiting EN Norm | Required Efficacy for pass | Minimum organisms to be tested and strain number |
|---|---|---|---|
| Bactericidal | EN13727 | >3 log (99.9%) (under dirty conditions within 60 s) | *Enterococcus hirae* ATCC 10541 - gram positive<br>ATCC 6538 - gram positive *Pseudomonas aeruginosa* ATCC 15442<br>- gram negative *Escherichia coli* K12 NCTC 10538 |
| Yeasticidal | EN13624 | >2 log (99%) (under dirty conditions within 60 s) | *Candida albicans* DSM 1386 |

The minimum organism to be tested in EN1499 is *Escherichia coli* K12 NCTC 10538.

In the present disclosure we refer to European standard methods however, similar test methods may be available outside Europe with different name of code but with the same or similar purpose.

**Counting and calculation of log reductions**

Limits of detection (LOD):

Depending on the dilution parameters of a method and the techniques used to enumerate cells, specific limits of detection must be set to ensure reliable enumeration.

Standard throughput (pour plate) - For counting of pour plates, colonies from the incubated plates were enumerated using the lower count limit of <10 and the upper count limit of >330. For example, if a plate had 7 visible colonies countable, <10 would be recorded and 10 would be used for the calculation. For the upper limit, if 407 colonies were counted on a plate, >330 would be recorded and 330 would be used for the calculation. Where the observed logarithmic reduction (log-kill) of microorganism colony-forming units (CFU) is greater than the limit of detection a greater-than ">", or less than "<" value would be used.

When reporting the final log reduction value, if a lower limit of detection has been used to make the calculation

a ">" value will be reported for example ">5.02 log reduction", while if upper limit of detection has been used to make the calculation a "<" value will be reported for example "<2.92 log reduction".

**Enumeration and calculation of N (Test organism suspension)**

Standard throughput (pour plate)-

$$N = \frac{(C \times df)}{100}$$

Where

*C* is the sum of viable count values
*df* is the dilution factor corresponding to dilution

Example:

$$N = \frac{((167+213) \times 10^7)}{100} = \frac{190 \times 10^7}{100} = 1.9 \times 10^7 \text{ cfu/ml}$$

**Enumeration and calculation of *T* (Test mixture)**

Standard throughput (pour plate)-

First, the mean average of cfu per 1 mL aliquot was established using the below calculation;

$$T = \left(\frac{d_1}{2}\right)$$

Where

$d_1$ is sum of viable count values from duplicate plates

Example:

$$T = \frac{308+292}{2} = \frac{600}{2} = 300 \text{ cfu/mL}$$

During the neutralization step, the test mixture underwent a number of dilutions. To account for this a final multiplication is applied;
For the $10^{-1}$ plate-

$$T = 300 \times 10 = 3000 = 3.0 \times 10^3 \text{ cfu/mL}$$

**Log reduction calculations (*N*_7)**

Before calculating the log reduction both N and T were converted into a logarithm base 10 value.
For example;

$$N_= 1.9 \times 10^7 = 7.28 \log 10$$

$$T = 3.0 \times 10^3 = 3.48 \log 10$$

To calculate the final log reduction the following calculation was used;

$$N - T = Log\ Reduction$$

For example;

$$7.28 - 3.48 = 3.80$$

The final log reduction for this example is 3.80

**Results**

Table 1 provides several example or reference formulations prepared in accordance with the disclosure. Each of the formulations of table 1 has a composition that includes an amount of the ingredient on the left expressed as a percentage based on the total weight of the composition. All percentages by weight indicated herein are based upon the percent active composition.

The reference compositions in Table 1 were tested according to the European suspension test EN 13727 against *pseudomonas aeruginosa* (ATCC), *staphylococcus aureus* (ATCC), *Escherichia coli* (ATCC), and *Enterococcus hirae* (ATCC) and the European suspension test EN 13624 against *Candida albicans* (DSM 1376). All samples passed EN 13727, with all formulations passing microbial efficacy against bacteria with a contact time of 30 seconds with *pseudomonas aeruginosa* being the limiting bacteria. With a pass criteria of Log 2, all formulations passed EN 13624 against *Candida albicans* with a 30 second contact time. In comparison to the high logarithmic reductions (> log 5) against bacteria, candida was determined as the limiting organism against the skin disinfectant compositions.

Table 1. Example formulations.

| Table 1 | | |
|---|---|---|
| | REF1 | REF 2 |
| Demineralised Water | Quantum satis (q.s.) | q.s. |
| wt%Glycerin | 5.00 | 5.00 |
| wt% 2-Phenoxyethanol | 0.50 | 0.90 |
| wt% Didecyldimethylammonium Chloride | 0.55 | 0.55 |
| wt% Polysorbate 20 | 0.90 | 2.70 |
| wt% Lauryldimethylamine Oxide | 0.35 | |
| wt% Trisodium dicarboxymethyl alaninate (MGDA-Na3) | 0.10 | 0.10 |
| wt% Hydroxyethylcellulose | 0.50 | - |
| Hydrochloric Acid | q.s. | q.s. |
| Sodium Hydroxide | q.s. | q.s. |
| pH | 5.5 | 5.5 |
| Suspension testing, 30 seconds contact time<br>( >2 Log reduction required for pass vs *candida albicans*)<br>( >3 Log reduction required for pass vs *pseudomonas aeruginosa, Staphylococcus aureus, Escherichia coli, Enterococcus hirae*) | | |
| Log reduction *candida albicans* | **4.48** | **3.02** |
| Log reduction *pseudomonas aeruginosa* | **5.46** | **>3.38** |
| Log reduction *staphylococcus aureus* | **5.42** | **>3.38** |
| Log reduction *Escherichia coli* | **>5.51** | **>3.38** |
| Log reduction *Enterococcus hirae* | **>5.51** | **>3.38** |

Results of the yeasticidal suspension testing upon the described compositions carried out following the European testing norms EN 13624 against *Candida albicans* (DSM 1376) indicates that this is the limiting organism with regards to antimicrobial efficacy for the described skin disinfectant compositions. Thus all following examples were tested using the yeasticidal suspension test EN13624 in order to determine the efficacy of the composition. It is determined that for the described compositions the European norm EN 13624 is the limiting test for determining their antimicrobial efficacy.

The results of the bacteriacidal suspension test carried out for hand disinfectant compositions, following the European testing norms EN 13727, indicated that the *pseudomonas aeruginosa* (ATCC), *staphylococcus aureus* (ATCC) organisms are the two most limiting bacteriacidal organisms. Selected compositions in the following examples were also tested against these strains to further exemplify the efficacy spectrum of the described compositions.

Furthermore, the above reference compositions were tested according to the stringent European standard washing norm EN 1499 and passed with a 30 s exposure time. The results clearly show that a composition according to the invention, which holds no hazard classifications according to CLP regulations. All components were taken into consideration for classification for this assessment. A composition according to the disclosure is capable of meeting the stringent requirements of EN 13624, EN 16727 and EN 1499 required for a skin disinfectant product.

Table 2; * - denotes "not according to the present invention"

| | 1* | 2* | 3* | 4* | 5* | 6* | 7* | 8* | 9* | 10* | 11* | 12 |
|---|---|---|---|---|---|---|---|---|---|---|---|---|
| Demineralised Water | q.s. | q.s. | q.s. | q.s. | q.s. | q.s. | q.s. | q.s. | q.s. | q.s. | q.s. | q.s. |
| wt% Glycerin | 5.00 | 5.00 | 5.00 | 5.00 | 5.00 | 5.00 | 5.00 | 5.00 | 5.00 | 5.00 | - | 5.00 |
| wt% Dipropylene Glycol | - | - | - | - | - | - | - | - | - | - | 5.00 | - |
| wt% Didecyldimethyl ammonium Chloride | - | - | - | 0.30 | 0.45 | 0.90 | 0.30 | 0.45 | 0.90 | 0.48 | 0.48 | 0.55 |
| wt% Lactic acid | 0.72 | 0.72 | 0.72 | 0.72 | 0.72 | 0.72 | 0.72 | 0.72 | 0.72 | - | - | 0.90 |
| wt% Hydrogen peroxide | 2.00 | 4.00 | 8.00 | 2.00 | 2.00 | 2.00 | 2.00 | 2.00 | 2.00 | - | - | 2.00 |
| wt% 2-phenoxyethanol | 0.90 | 0.90 | 0.90 | 0.90 | 0.90 | 0.90 | 0.90 | 0.90 | 0.90 | 0.90 | 0.90 | 0.90 |
| wt% Polyglyceryl-10 Caparylate/Caprate | - | - | - | - | - | - | - | - | - | - | - | 2.70 |
| wt% Marlinat 242-70 | 0.55 | 0.55 | 0.55 | 0.55 | 0.55 | 0.55 | - | - | - | - | - | - |
| wt% Cocoamido propylbetaine | 0.90 | 0.90 | 0.90 | 0.90 | 0.90 | 0.90 | - | - | - | - | - | - |
| wt% Lauryldimethylamine oxide | 0.90 | 0.90 | 0.90 | 0.90 | 0.90 | 0.90 | - | - | - | - | - | - |
| wt% Myristyldimethylamine oxide | 0.90 | 0.90 | 0.90 | 0.90 | 0.90 | 0.90 | - | - | - | - | - | - |
| wt% Trisodium dicarboxymethyl alaninate (MGDA-Na3) | 0.04 | 0.04 | 0.04 | 0.04 | 0.04 | 0.04 | 0.04 | 0.04 | 0.04 | 0.04 | 0.04 | 0.04 |
| pH | 3.9 | 3.9 | 3.9 | 3.9 | 3.9 | 3.9 | 2.3 | 2.3 | 2.3 | n/a | n/a | 2.6 |
| Suspension testing, average of 3 repeats. ( >2 log reduction required for pass vs candida albicans). | | | | | | | | | | | | |
| Log reduction *candida albicans (30 sec*) | -0.02 | 0.41 | 1.65 | 0.52 | 0.59 | 0.65 | **>3.43** | **>3.43** | **>3.43** | **3.37** | **3.41** | **>3.38** |
| Log reduction *candida albicans* (*60 sec*) | 0.03 | 0.59 | 3.17 | - | - | - | **>3.43** | **>3.43** | **>3.43** | **3.37** | **3.41** | **>3.38** |

The series of formulations of table 2, numbered 1-12 have been prepared and tested for antimicrobial efficacy according to the EN 13624 suspension test. The efficacy results have been calculated on a log scale. For EN 13624 a 2 log reduction in colony-forming units (CFU) is required for a pass. In percentages this is equivalent to a reduction in CFU of 99%.

Compositions 1 through to 3 are not prepared according to the invention. These hydrogen peroxide containing compositions do not contain quaternary ammonium compounds and thus do not show efficacy at 60 seconds in a EN 13624 study at concentrations of less than 8 wt % hydrogen peroxide. Furthermore, since hydrogen peroxide is being used as the primary antimicrobial active for these compositions, said compositions must contain a hydrogen peroxide stabilizer (i.e. lactic acid) and must be formulation at a low pH (i.e. <pH4) to maintain stability.

Compositions 4-6 include didecyldimethylammonium chloride at increasing concentrations up to 1 wt% active. Compositions 4-6 have same components and amounts of composition 1 with the exception of the presence of didecyldimethylammonium choride. The inclusion of didecyldimethylammonium chloride does show an improvement in efficacy relative to composition 1 (with same amount of H2O2) however are are not prepared according to the invention. They contain 0.55 wt% of an anionic surfactant (Marlinat 242-70, sodium laureth sulfate) and they do not show the required 2 log efficacy in a EN 13624 study. A formulation containing an aromatic alcohol, hydrogen peroxide and didecyldimethyl ammonium chloride as actives with a surfactant base consisting of a mixture of anionic and amphoteric surfactants (sodium laureth sulfate, cocoamidopropyl betaine, lauryldimethylamine oxide, and myristyldimethylamine oxide) (4-6) can have its microbial efficacy against *candida albicans* increased by significantly reducing the concentration level of the mixture of anionic and amphoteric surfactants or by substituting the surfactant mixture with a non-ionic surfactant as seen in composition 12. Composition 12 has been prepared according to the invention.

Compositions 7-11 are compositions essentially free of surfactant, disregarding quaternary ammonium compounds (didecyldimethylammonium chloride). Compositions 7-11, shows how removal of the surfactant from a formulation significantly improves efficacy, whilst compositions 10, 11 show that quaternary ammonium compounds demonstrate the required efficacy in such a system in the absence of hydrogen peroxide and lactic acid. These compositions are not are not prepared according to the invention and it is known in the art that surfactants are required for aesthetics (skin feel, foaming) and cleansing in hand disinfectant applications. The disinfectant effect of a quaternary ammonium compound such as didecyldimethylammonium chlodride can therefore been shown in Table 2. When didecyldimethylammonium chlodride used alongside a selection of surfactants, efficacy is hindered. Compositions 7-11 demonstrate the influencing role of surfactants in inhibiting biocidal efficacy and demonstrate the importance of the surfactant types, concentrations and combinations when formulating a skin disinfectant product which demonstrates the required antimicrobial efficacy.

Table 3

| | 18 | 19 | 22 | 23 |
|---|---|---|---|---|
| Demineralised Water | q.s. | q.s. | q.s. | q.s. |
| wt% Glycerin | 5.00 | 5.00 | 5.00 | 5.00 |
| wt% Polyglyceryl-10 caprylate/caprate | 2.70 | - | - | - |
| wt% Polysorbate 20 | - | 2.70 | - | - |
| wt% Cocoamidopropyl betaine | - | - | 0.42 | - |
| wt% Lauryldimethylamine oxide | - | - | 0.42 | 1.35 |
| wt% Myristyl dimethyl amine ocide | - | - | 0.42 | 1.35 |
| wt% Didecyldimethylammonium Chloride | 0.55 | 0.55 | 0.55 | 0.55 |
| wt% 2-phenoxyethanol | 0.90 | 0.90 | 0.90 | 0.90 |
| wt% Trisodium dicarboxymethyl alaninate (MGDA-Na3) | 0.10 | 0.10 | 0.04 | 0.04 |
| Hydrochloric Acid | qs | qs | - | - |
| Sodium Hydroxide | qs | qs | - | - |
| pH | 5.5 | 5.5 | 10.2 | 8.6 |
| Suspension testing, 30 seconds contact time<br>Log reduction<br>( >2 Log reduction required for pass vs *candida albicans*)<br>( >3 Log reduction required for pass vs *pseudomonas aeruginosa*)<br>( >3 Log reduction required for pass vs *staphylococcus aureus*) | | | | |
| Log reduction *candida albicans* (*30 seconds)* | **2.44** | **3.02** | 1.11 | 1.69 |
| Log reduction *candida albicans* (*60 seconds)* | | | **2.85** | **3.09** |

(continued)

| Table 3 | | | | |
|---|---|---|---|---|
| | 18 | 19 | 22 | 23 |
| Log reduction *pseudomonas aeruginosa* (*30 seconds*) | **>3.38** | **>3.38** | - | - |
| Log reduction *staphylococcus aureus* (*30 seconds*) | **>3.38** | **>3.38** | - | - |

The series of compositions in table 3, numbered 18, 19, 22, and 23 are prepared according to the invention. Compositions 18 and 19 include 2.70 wt% non-ionic surfactant, showing the required 2 log reduction in CFU pass level for EN 13624 in a 60 second contact time, and additionally showing the required pass level in a 30 second contact time. Additionally, compositions 18 and 19 were tested against *pseudomonas aeruginosa* with a 30 second contact time according to the EN 13727 bacteriacidal suspension testing conditions (compositions 18, 19 also tested against *staphylococcus aureus* under the same conditions). These compositions demonstrated the required 3 log reduction in CFU pass level for EN 13727.

Compositions 22 and 23 demonstrate the required 2 log reduction in CFU pass level for EN 13624 in a 60 second contact time with up to 3 wt% amphoteric surfactant (2.70 wt%), satisfying the yeasticidal efficacy requirements for a antimicrobial hand disinfectant.

Efficacy was found to be significantly increased by use of a non-ionic surfactant such as polyglyceryl esters (polyglyceryl-10 caprylate/caprate) and polysorbate (polysorbate-20) chemistries. The required efficacy according to EN 13624 could be achieved with up to 5 wt% non-ionic surfactant in a 60 second contact time or greater than 3 wt% in a 30 second contact time. Compositions 18, 19, 22, and 23 also show that efficacy can be achieved over a broad pH spectrum ranging from 5.5 to >10.2.

| Table 4 | | | | | | | | |
|---|---|---|---|---|---|---|---|---|
| | 24a | 24b | 25 | 26 | 27 | 28 | 29 | 30 |
| Demineralised Water | q.s. | q.s. | q.s. | q.s. | q.s. | q.s. | q.s. | q.s. |
| wt% Glycerin | 5.00 | 5.00 | 5.00 | 5.00 | 5.00 | 5.00 | 5.00 | 5.00 |
| wt% Polysorbate 20 | 2.00 | 1.30 | 2.00 | 1.30 | 1.30 | 1.30 | 0.90 | 0.90 |
| wt% Didecyldimethyl ammonium Chloride | 0.55 | 0.55 | 0.55 | 0.55 | 0.55 | 0.55 | 0.55 | 0.55 |
| wt% 2-phenoxyethanol | 0.90 | 0.90 | 0.90 | 0.90 | 0.90 | 0.90 | 0.90 | 0.90 |
| wt% Lauryldimethylamine oxide | 0.21 | 0.21 | 0.21 | 0.21 | 0.70 | 0.70 | 0.90 | 0.90 |
| wt% Trisodium dicarboxymethyl alaninate (MGDA-Na3) | 0.10 | 0.10 | 0.10 | 0.10 | 0.10 | 0.10 | 0.10 | 0.10 |
| wt% Hydroxyethylcellulose | 0.50 | 0.50 | 0.50 | 0.50 | 0.50 | 0.50 | 0.50 | 0.50 |
| wt% Hydrochloric Acid (1 mol) | 0.69 | 0.69 | - | 0.06 | 1.44 | 1.26 | 1.51 | 1.58 |
| wt% Sodium Hydroxide (1 mol) | 0.01 | 0.01 | 0.24 | 0.36 | 0.23 | 0.83 | | 1.09 |
| pH | 5.5 | 5.5 | 7.0 | 4.0 | 5.5 | 7.0 | 5.5 | 7.0 |
| Suspension testing results, 30 seconds contact time (>2 Log reduction required for pass) | | | | | | | | |
| Log reduction *candida albicans* | **2.01** | **2.95** | **2.51** | **2.67** | **2.38** | **2.50** | **2.65** | **2.49** |

The series of compositions in table 4, numbered 24-30 are prepared according to the invention. Compositions 24 through to 30 all include a combination of non-ionic and amphoteric surfactants, notably polysorbate 20 and lauryldimethylamine oxide respectively, with a total (non-biocidal, cf. didecyldimethylammonium chloride) surfactant level of greater than 2 wt%.

Compositions 24-30 all show the required 2 log reduction in CFU pass level for EN 13624 in a 30 second contact time, showing remarkable efficacy in a short contact time. Compositions 24 through to 26 additionally hold no hazard classifications according to CLP regulations.

The role of 2-phenoxyethanol in improving efficacy is a novel discovery and an essential component of the invention. Those in the art could compare the above composition to similar compositions where the level of surfactant, type of surfactant and biocide used is such that the inclusion or non-inclusion of phenoxyethanol would dictate whether said composition would demonstrate the desired antimicrobial efficacy for an antimicrobial skin disinfectant composition.

Table 5

| | 31 | 32 | 33 | 34 | 35 | 36 | 37 |
|---|---|---|---|---|---|---|---|
| Demineralised Water | q.s. | q.s. | q.s. | q.s. | q.s. | q.s. | q.s. |
| wt% Glycerin | 5.00 | 5.00 | 5.00 | 5.00 | 5.00 | 5.00 | 5.00 |
| wt% Polyglyceryl-10 caprylate/caprate | 0.90 | - | 0.90 | 0.70 | 1.30 | - | 0.90 |
| wt% Polysorbate 20 | - | 1.30 | - | - | - | 1.30 | - |
| wt% Didecyldimethyl ammonium Chloride | 0.55 | 0.55 | 0.55 | 0.55 | 0.55 | 0.55 | 0.55 |
| wt% 2-phenoxyethanol | 0.90 | 0.90 | 0.90 | 0.90 | 0.90 | 0.50 | 0.50 |
| wt% Decyldimethyl amine oxide | 0.90 | 0.90 | - | 0.70 | - | - | - |
| wt% Lauryldimethyl amine oxide | - | - | 0.90 | 0.70 | 0.70 | 0.35 | 0.35 |
| wt% Trisodium dicarboxymethyl alaninate (MGDA-Na3) | 0.10 | 0.10 | 0.10 | 0.10 | 0.10 | 0.10 | 0.10 |
| wt% Hydroxyethyl cellulose | 0.75 | 0.75 | 0.75 | 0.50 | 0.50 | 0.50 | 0.50 |
| wt% Hydrochloric Acid (1 mol) | 1.54 | 1.51 | 1.32 | - | 0.97 | 0.83 | 1.05 |
| wt% Sodium Hydroxide (1 mol) | - | - | - | 0.37 | 0.52 | 0.05 | 0.27 |
| pH | 5.5 | 5.5 | 5.5 | 5.5 | 7.0 | 5.5 | 5.5 |
| Suspension testing results, 30 seconds contact time (>2 Log reduction required for pass) | | | | | | | |
| Log reduction *candida albicans* | **>3.72** | **>3.72** | **2.48** | **2.94** | **2.13** | **3.55** | **3.29** |
| Log reduction *pseudomonas aeruginosa* | **>4.99** | **5.27** | **5.50** | - | - | **5.36** | **>5.51** |
| Log reduction *staphylococcus aureus* | **>5.54** | **>5.54** | **>5.54** | - | - | **5.22** | **5.49** |

The series of compositions in table 5, numbered 31-37 are prepared according to the invention. Compositions 31 through to 37 include a broader combination of non-ionic and amphoteric surfactants, compared to table 4, with a total (non-biocidal, cf. didecyldimethylammonium chloride) surfactant level of greater than 1 wt%.

Compositions 31-37 all show the required 2 log reduction in CFU pass level for EN 13624 in a 30 second contact time, showing remarkable efficacy in a short contact time. Selected results are also seen against *pseudomonas aeruginosa* and *staphylococcus aureus* with a 30 second contact time according to the EN 13727 bacteriacidal suspension testing conditions. These compositions demonstrated the required 3 log reduction in CFU pass level for EN 13727. Compositions 36 and 37 additionally hold no hazard classifications according to CLP regulations.

Table 6

| | 38 | 39 | 40 | 41 | 42 | 43 | 44 | 45 |
|---|---|---|---|---|---|---|---|---|
| Demineralised Water | q.s. | q.s. | q.s. | q.s. | q.s. | q.s. | q.s. | q.s. |
| wt% Glycerin | 5.00 | 5.00 | 5.00 | 5.00 | 5.00 | 5.00 | 5.00 | 5.00 |
| wt% Didecyldimethylammonium Chloride | 0.55 | 0.55 | 0.55 | 0.55 | 0.55 | 0.55 | 0.55 | 0.55 |
| wt% Polysorbate 20 | 1.00 | - | - | - | - | - | - | - |
| wt% β -Alanine, N-(2-carboxyethyl) N-coco alkyl derivative | - | - | - | 2.50 | - | - | - | - |
| wt% Polyethylene glycol (23) lauryl ether | - | - | - | - | 2.50 | - | - | - |
| wt% Polyoxyethylene (21) stearyl ether | - | - | - | - | - | 2.50 | - | - |
| wt% Decyl glucoside | - | - | - | - | - | - | 1.00 | - |
| wt% Cocamidopropyl betaine | - | - | - | - | - | - | - | 1.00 |
| wt% Lauryldimethylamine oxide | 2.00 | 2.00 | 3.00 | - | - | - | - | - |
| wt% 2-phenoxyethanol | 0.90 | 0.90 | 0.90 | 0.90 | 0.90 | 0.90 | 0.90 | 0.90 |
| wt% trisodium salt of methylglycinediacetic acid (Na3MGDA) | 0.10 | 0.10 | 0.10 | 0.10 | 0.10 | 0.10 | 0.10 | 0.10 |
| Hydrochloric Acid | qs | qs | qs | qs | qs | qs | qs | qs |
| Sodium Hydroxide | qs | qs | qs | qs | qs | qs | qs | qs |
| pH | 5.5 | 5.5 | 5.5 | 5.5 | 5.5 | 5.5 | 5.5 | 5.5 |
| Suspension testing results, 60 seconds contact time (>2 Log reduction required for pass) | | | | | | | | |

(continued)

| Table 6 | | | | | | | | |
|---|---|---|---|---|---|---|---|---|
| | 38 | 39 | 40 | 41 | 42 | 43 | 44 | 45 |
| Log reduction *Candida albicans (60 seconds)* | **2.10** | **2.21** | **2.09** | **>3.39** | **3.21** | **>3.39** | **>3.39** | **2.37** |

The series of compositions in Table 6 numbered 38 to 45 are prepared according to the disclosure and formulated using a range of different nonionic and amphoteric surfactants singularly and in combination; this list of surfactants that can be used in a skin disinfectant composition made according to the invention, include, but are not limited to ethoxylated sorbitan esters (c.f polysorbates), ß-Alanine, N-(2-carboxyethyl) N- alkyl derivatives or salts, polyethylene glycol alkyl ethers (c.f. alcohol ethoxylates), alkyl glucosides and alkyl polyglucosides, polyglyceryl esters, amine oxides, Cocamidopropyl betaine (CAPB) or coco betaine and similar chemistries. All formulations exceeded the pass criteria using a 60 s contact time against *Candida albicans* according to the European standard yeastercidal suspension test EN 13624.

Table 7

| | REF1 | 46 | 47 | 48 | 49 | 50 | 51 | 52 | 53 | 54 | 55 | 56 | 57 | 58 |
|---|---|---|---|---|---|---|---|---|---|---|---|---|---|---|
| Demineralised Water | q.s. | q.s. | q.s. | q.s. | q.s. | q.s. | q.s. | q.s. | q.s. | q.s. | q.s. | q.s. | q.s. | q.s. |
| wt% Glycerin | 5.00 | 5.00 | 5.00 | 5.00 | 5.00 | 5.00 | 5.00 | 5.00 | 5.00 | 5.00 | 5.00 | 5.00 | 5.00 | 5.00 |
| wt% Didecyldimethylammonium Chloride | 0.55 | - | - | 0.55 | 0.55 | 0.55 | 0.55 | 0.55 | 0.55 | 0.55 | 0.55 | 0.55 | 0.55 | 0.55 |
| wt% Dioctylldimethylammonium Chloride | - | 0.55 | - | - | - | - | - | - | - | - | - | - | - | - |
| wt% C8-C8, C8-C10, C10-C10 dimethylammonium Chloride | - | - | 0.55 | - | - | - | - | - | - | - | - | - | - | - |
| wt% Cetrimonium Chloride | - | - | - | 1.00 | - | - | - | - | - | - | - | - | - | - |
| wt% Cetyl Trimethyl Ammonium Chloride | - | - | - | - | 1.00 | - | - | - | - | - | - | - | - | - |
| wt% Polysorbate 20 | 2.70 | 2.00 | 2.00 | - | - | - | - | - | - | - | - | - | - | - |
| wt% Polysorbate 80 | - | - | - | - | - | 2.00 | - | - | - | - | - | - | - | - |
| wt% Polyethylene glycol (23) lauryl ether | - | - | - | - | - | - | 1.00 | - | - | - | - | - | - | - |
| wt% C9-11 hexaethylene glycol | - | - | - | - | - | - | - | 1.00 | - | - | - | - | - | - |
| wt% Polyoxyethylene (21) stearyl ether | - | - | - | - | - | - | - | - | 1.00 | - | - | - | - | - |
| wt% $\beta$ -Alanine, N-(2-carboxyethyl) N-coco alkyl derivative | - | - | - | - | - | - | - | - | - | 1.00 | - | - | - | - |
| wt% Octyldimethylamine oxide | - | - | - | - | - | - | - | - | - | - | 1.00 | - | - | - |
| wt% Decyldimethylamine oxide | - | - | - | - | - | - | - | - | - | - | - | 1.00 | - | - |
| wt% Lauryldimethylamine oxide | - | - | - | - | - | - | - | - | - | - | - | - | 1.00 | - |
| wt% Myristyldimehtylamine oxide | - | - | - | - | - | - | - | - | - | - | - | - | - | 1.00 |
| wt% 2-phenoxyethanol | 0.90 | 0.90 | 0.90 | 0.90 | 0.90 | 0.90 | 0.90 | 0.90 | 0.90 | 0.90 | 0.90 | 0.90 | 0.90 | 0.90 |
| wt% trisodium salt of methylglycinediacetic acid (Na3MGDA) | 0.10 | 0.10 | 0.10 | 0.10 | 0.10 | 0.10 | 0.10 | 0.10 | 0.10 | 0.10 | 0.10 | 0.10 | 0.10 | 0.10 |
| Hydrochloric Acid | qs | qs | qs | qs | qs | qs | qs | qs | qs | qs | qs | qs | qs | qs |
| Sodium Hydroxide | qs | qs | qs | qs | qs | qs | qs | qs | qs | qs | qs | qs | qs | qs |
| pH | 5.5 | 5.5 | 5.5 | 5.5 | 5.5 | 5.5 | 5.5 | 5.5 | 5.5 | 5.5 | 5.5 | 5.5 | 5.5 | 5.5 |
| Suspension testing results, 30 seconds contact time (>2 Log reduction required for pass) | | | | | | | | | | | | | | |
| Log reduction *candida albicans* | **3.02** | **>3.51** | **>3.51** | **>4.80** | **>4.80** | **2.72** | **>4.81** | **0 3.86** | **3.76** | **>4.80** | **2.31** | **2.11** | **2.25** | **4.80** |
| Log reduction *Pseudomonous aeruginosa* | **>3.38** | **>6.75** | **6.06** | **>5.87** | **>5.87** | **4.21** | **>5.87** | **4.92** | **4.82** | **4.64** | **>5.87** | **>5.87** | **>5.87** | **>5.87** |

The series of compositions in Table 7 numbered 46, 47, and 50 to 58 are prepared according to the invention. Compositions were prepared using the same base ingredients (humectant, chelator, aromatic alcohol, pH modifiers and water) with different quartenary ammonium compounds, nonionic surfactants and amphoteric surfactants. Compositions 38 through to 50 include a range of quaternary ammonium compounds including Didecyldimethylammonium Chloride, Dioctylldimethylammonium Chloride, mixtures of di-Ca, $C_8$-$C_{10}$, di-$C_{10}$ dimethylammonium Chloride, Cetrimonium Chloride, Cetyl Trimethyl Ammonium Chloride used singularly and in combination. All quaternary ammonium based compounds exceeded the required pass criteria using a 30 second contact time against candida albicans (> 2 log reduction) according to the European standard yeastercidal suspension test EN 13624 and against *pseudomonas aeruginosa* (> 3 log reduction) according to the European standard bacteriacidal suspession test EN 13727.

Also following the invention, compositions 46, 47 and 50 through to 54 include a range of nonionic surfactants. All compositions showed high yeastercidal and bacteriacidal efficacy, exceeding the required pass criteria using a 30 second contact time against candida albicans (> 2 log reduction) according to the European standard yeastercidal suspension test EN 13624 and against *pseudomonas aeruginosa* (> 3 log reduction) according to the European standard bacteriacidal suspension test EN 13727.

Compositions 55 through to 58 were made with a range of different amphoteric surfactants according to the invention. Compositions 55 to 58 all exceeded the required pass criteria using a 30 second contact time against candida albicans (> 2 log reduction) according to the European standard yeastercidal suspension test EN 13624 and against *pseudomonas aeruginosa* (> 3 log reduction) according to the European standard bacteriacidal suspension test EN 13727.

Compositions 48 and 49 include cationic quaternary ammonium compounds which themselves do not demonstrate antimicrobial efficacy. Though not required for disinfectant compositions of the disclosure, cationic surfactant including non-biocidal quaternary ammonium compounds can be conveniently used to provide users with a conditioning skin feel on the skin following the application of the product, thus ensuring more pleasing sensory benefits to the user after application and improving compliance.

Table 8; * - denotes "not according to the present invention"

(amounts in wt%)

| | 59 | 60* | 61 | 62 | 63 | 64 | 65 | 66 | 67 | 68 | 69 |
|---|---|---|---|---|---|---|---|---|---|---|---|
| Demineralised Water | q.s. | q.s. | q.s. | q.s. | q.s. | q.s. | q.s. | q.s. | q.s. | q.s. | q.s. |
| Glycerin | 5.00 | 5.00 | 5.00 | 5.00 | 5.00 | 5.00 | 5.00 | 5.00 | 5.00 | 5.00 | 5.00 |
| Polysorbate 20 | 2.01 | 2.01 | 2.00 | 2.00 | 2.70 | 2.50 | 2.30 | 2.10 | 1.90 | 1.70 | 1.50 |
| Bardac 208M (mixture of C8-C8, C8-C10, C10-C10 dimethylammonium chloride & Alkyl (C12-16) dimethylbenzyl ammonium chloride (ADBAC, BKC)), 80% active in water | 0.66 | - | - | - | - | - | - | - | - | - | - |
| Barquat LB50 (Alkyl (C12-C14) dimethylbenzylammonium chloride, (ADBAC, BKC)), 50% active in water | - | 1.10 | - | - | - | - | - | - | - | - | - |
| Bardac 2080 (mixture of C8-C8, C8-C10, C10-C10 dimethylammonium chloride), 80% in water | - | - | 0.67 | - | 0.67 | 0.67 | 0.67 | 0.67 | 0.67 | 0.67 | 0.67 |
| Barquat DM50 (Alkyl (C12-16) dimethylbenzyl ammonium chloride (ADBAC, BKC)), 50% active in water | - | - | - | 1.11 | - | - | - | - | - | - | - |
| Phenoxyethanol | 0.90 | 0.90 | 0.90 | 0.90 | 0.90 | 0.90 | 0.90 | 0.90 | 0.90 | 0.90 | 0.90 |
| Trilon M (40% active in water) | 0.25 | 0.25 | 0.27 | 0.27 | 0.25 | 0.25 | 0.25 | 0.25 | 0.25 | 0.25 | 0.25 |
| Hydrochloric Acid (1 mol) | q.s. | q.s. | q.s. | q.s. | q.s. | q.s. | q.s. | q.s. | q.s. | q.s. | q.s. |
| Sodium Hydroxide (1 mol) | q.s. | q.s. | q.s. | q.s. | q.s. | q.s. | q.s. | q.s. | q.s. | q.s. | q.s. |
| pH | 5.5 | 5.5 | 5.5 | 5.5 | 5.5 | 5.5 | 5.5 | 5.5 | 5.5 | 5.5 | 5.5 |
| EN13624 Suspension testing results, 30 seconds contact time (>2 log reduction required for pass) | | | | | | | | | | | |
| Log reduction *candida albicans* | **3.14** | **2.97** | **4.36** | **2.15** | **2.35** | **2.71** | **2.92** | **3.03** | **3.07** | **3.19** | **3.53** |

The series of compositions in Table 8 numbered 59 and 61 to 69 are prepared according to the invention. The compositions were prepared using polysorbate 20 as non-ionic surfactant and phenoxyethanol as aromatic alcohol with different quartenary ammonium compounds. Composition 59 includes as quaternary ammonium compounds Bardac 208M (mixture of C8-C8, C8-C10, C10-C10 dimethylammonium chloride & alkyl (C12-16) dimethylbenzyl ammonium chloride (ADBAC, BKC)). Composition 60 includes as quaternary ammonium compounds Barquat LB50 (Alkyl (C12-C14) dimethylbenzylammonium chloride, (ADBAC, BKC)). Compositions 61 and 63 to 69 include as quaternary ammonium compounds Bardac 2080 (mixture of C8-C8, C8-C10, C10-C10 dimethylammonium chloride.

Composition 62 includes as quaternary ammonium compounds Barquat DM50 (Alkyl (C12-16) dimethylbenzyl ammonium chloride (ADBAC, BKC)). All quaternary ammonium based compounds exceeded the required pass criteria using a 30 second contact time against candida albicans (> 2 log reduction) according to the European standard yeastercidal suspension test EN 13624.

Table 9

(amounts in wt%)

| | 70 |
|---|---|
| Demineralised Water | q.s. |
| Glycerin | 5.00 |
| Polysorbate 20 | 2.70 |
| Bardac 2080 (mixture of C8-C8, C8-C10, C10-C10 dimethylammonium chloride), 80% in water | 0.68 |
| Phenoxyethanol | 0.90 |
| Trilon M (40% active in water) | q.s. |
| Hydrochloric Acid (1 mol) | q.s. |
| pH | 5.5 |
| EN13624 suspension testing results, (>2 log reduction required for pass) | |
| *candida albicans (EN13624) 15s contact time* | **2.22** |

Table 9 shows composition 70 according to the invention. In composition 70, Bardac 2080 (mixture of C8-C8, C8-C10, C10-C10 dimethylammonium chloride) was used as quaternary ammonium compound. Composition 70 achieved the required pass criteria against *candida albicans* (> 2 log reduction) according to the European standard yeastercidal suspension test EN 13624 even after 15s contact time.

Table 10; * - denotes "not according to the present invention"

(amounts in wt%)

| | 71* |
|---|---|
| Demineralised Water | q.s. |
| Glycerin | 5.00 |
| Lonzagard BKC cGMP (Alkyl (C12-18) dimethylbenzyl ammonium chloride, (ADBAC)), 50% in water | 0.26 |
| Polyglyceryl-10 Caprylate/Caprate | 0.50 |
| Hydroxyethylcellulose | 0.50 |
| Phenoxyethanol | 0.90 |
| Trilon M (40% active in water) | 0.25 |
| Hydrochloric Acid (1 mol) | q.s. |
| Sodium Hydroxide (1 mol) | q.s. |
| pH | 5.5 |
| EN13624 suspension testing results, 30 seconds contact time (>2 log reduction required for pass) | |
| Log red. *candida albicans* | **2.02** |
| EN13727 suspension testing results, 30 seconds contact time (>3 log reduction required for pass) | |
| Log red. *pseudomonas aeruginosa* | **3.67** |

(continued)

| Table 10; * - denotes "not according to the present invention" | |
|---|---|
| (amounts in wt%) | |
| | 71* |
| Log red. *staphylococcus aureus* | **3.89** |

The composition in Table 10 numbered 71 is not prepared according to the invention. Polyglyceryl-10 caprylate/caprate was used as the non-ionic surfactant. Composition 71 exceeded the required pass criteria using a 30 second contact time against *candida albicans* (> 2 log reduction) according to the European standard yeastercidal suspension test EN 13624. Moreover, composition 71 exceeded the required pass criteria using a 30 second contact time against *pseudomonas aeruginosa and staphylococcus aureus* (> 3 log reduction) according to the European standard bactericidal suspension test EN 13727.

## Claims

**1.** A skin disinfectant composition comprising:

a. one or more quaternary ammonium compounds in an amount of from 0.1 to 2.0 wt% based upon the total weight of the composition, wherein said one or more quaternary ammonium compounds comprise a dimethyl dialkyl ammonium chloride wherein each alkyl group contains 8 to 12 carbon atoms;
b. one or more surfactants in an amount of from 0.1 to 4.0 wt% based upon the total weight of the composition, wherein said one or more surfactants are selected from the group consisting of non-ionic surfactants, amphoteric surfactants, and mixtures thereof,

wherein the non-ionic surfactant is selected from the group consisting of polyglyceryl-10 caprylate/caprate, polysorbate 20, polysorbate 80, decyl glucoside, polyethylene glycol (23) lauryl ether, C9-11 hexaethylene glycol alkyl ethers and polyoxyethylene (21) stearyl ether, and
wherein the amphoteric surfactant is selected from the group consisting of octyldimethyl amine oxide, decyldimethyl amine oxide, lauryldimethyl amine oxide, isoalkyl dimethyl amine oxide, tetradecyldimethyl amine oxide, cetyldimethyl amine oxide, cocoamidopropyl betaine and β-Alanine, N-(2-carboxyethyl)-, N-coco alkyl derivatives; and

c. an aromatic alcohol in an amount of from 0.1 to 1.0 wt% based upon the total weight of the composition, wherein the aromatic alcohol is phenoxyethanol, and
wherein the composition does not contain an anionic surfactant.

**2.** The composition of claim 1, wherein the aromatic alcohol is present in an amount of from 0.1 to 1.0 wt% based upon the total weight of the composition and wherein the one or more surfactants comprise at least one amphoteric surfactant present in an amount of from 0.1 to 4.0 wt%, based upon the total weight of the composition.

**3.** The composition of any one of the preceding claims, wherein the one or more surfactants comprise at least a non-ionic surfactant and at least an amphoteric surfactant.

**4.** The composition of any of the preceding claims, wherein the one or more quaternary ammonium compounds are present in an amount of 0.2 to 1.0 wt% based upon the total weight of the composition or wherein the one or more quaternary ammonium compounds are present in an amount of 0.2 to 0.6 wt% based upon the total weight of the composition.

**5.** The composition of any one of the preceding claims, wherein the one or more quaternary ammonium compounds comprise didecyldimethylammoniun chloride.

**6.** The composition of any one of the preceding claims,

(i) wherein the aromatic alcohol is present in an amount of from 0.1 to 0.5 wt% based upon the total weight of

the composition, wherein the one or more surfactants comprise at least a non-ionic surfactant and at least an amphoteric surfactant in an amount of from 1.0 to 4.0 wt% based upon the total weight of the composition and wherein the weight ratio of amphoteric surfactant to non-ionic surfactant is 1:1 to 1:6.

**7.** The composition of any one of the preceding claims, wherein the one or more quaternary ammonium compound is didecyldimethylammonium chloride.

**8.** The composition of any one of the preceding claims, wherein the one or more surfactants are selected from the group consisting of polysorbate 20, lauryl dimethyl amine oxide, polyglyceryl-10 caprylate/caprate, and mixtures thereof.

**9.** The composition of any one of the preceding claims, wherein the composition comprises

a. didecyldimethylammonium chloride in an amount of from 0.1 to 0.7 wt.% based upon the total weight of the composition;
b. one or more surfactants selected from polysorbate 20, lauryl dimethyl amine oxide, and polyglyceryl-10 caprylate/caprate in an amount of from 0.3 to 3.0 wt%, based upon the total weight of the composition; and

2-phenoxyethanol in an amount of from 0.1 to 1.0 wt%, based upon the total weight of the composition.

**10.** The composition of any of the preceding claims, further comprising a humectant, a solvent, a fragrance, a colouring agent or dye, an opacifying agent, a pearlizing agent, a vitamin, an antioxidant, an emollient, a skin care additive, a foaming agent, a polymeric thickener or mixtures thereof.

**11.** Hand soap in the form of a liquid, gel or foam comprising the composition of any of the preceding claims.

## Patentansprüche

**1.** Hautdesinfektionszusammensetzung, welche aufweist:

a. eine oder mehrere quartäre Ammoniumverbindungen in einer Menge von 0,1 bis 2,0 Gew.-% bezogen auf das Gesamtgewicht der Zusammensetzung, wobei die einen oder mehreren quartären Ammoniumverbindungen ein Dimethyldialkylammoniumchlorid umfassen, wobei jede Alkylgruppe 8 bis 12 Kohlenstoffatome aufweist;
b. ein oder mehrere Tenside in einer Menge von 0,1 bis 4,0 Gew.-% bezogen auf das Gesamtgewicht der Zusammensetzung, wobei die einen oder mehreren Tenside aus der Gruppe ausgewählt sind, die aus nichtionischen Tensiden, amphoteren Tensiden und Mischungen davon besteht,

wobei das nichtionische Tensid aus der Gruppe ausgewählt ist, die aus Polyglyceryl-10-Caprylat/Caprat, Polysorbat 20, Polysorbat 80, Decylglucosid, Polyethylenglycol(23)laurylether, C9-11-Hexaethylenglycolalkylether und Polyoxyethylen(21)stearylether besteht, und
wobei das amphotere Tensid aus der Gruppe ausgewählt ist, die aus Octyldimethylaminoxid, Decyldimethylaminoxid, Lauryldimethylaminoxid, Isoalkyldimethylaminoxid, Tetradecyldimethylaminoxid, Cetyldimethylaminoxid, Cocoamidopropyl-Betain und β-Alanin, N-(2-Carboxyethyl)-, N-Cocosalkyl-Derivaten besteht; und

c. einen aromatischen Alkohol in einer Menge von 0,1 bis 1,0 Gew.-% bezogen auf das Gesamtgewicht der Zusammensetzung, wobei der aromatische Alkohol Phenoxyethanol ist und wobei die Zusammensetzung kein anionisches Tensid enthält.

**2.** Zusammensetzung nach Anspruch 1, wobei der aromatische Alkohol in einer Menge von 0,1 bis 1,0 Gew.-% bezogen auf das Gesamtgewicht der Zusammensetzung vorliegt und wobei die einen oder mehreren Tenside mindestens ein amphoteres Tensid umfassen, das in einer Menge von 0,1 bis 4,0 Gew.-% bezogen auf das Gesamtgewicht der Zusammensetzung vorliegt.

**3.** Zusammensetzung nach einem der vorhergehenden Ansprüche, wobei die einen oder mehreren Tenside mindestens ein nichtionisches Tensid und mindestens ein amphoteres Tensid umfassen.

**4.** Zusammensetzung nach einem der vorhergehenden Ansprüche, wobei die einen oder mehreren quartären Ammoniumverbindungen in einer Menge von 0,2 bis 1,0 Gew.-% bezogen auf das Gesamtgewicht der Zusammensetzung vorliegen oder wobei die einen oder mehreren quartären Ammoniumverbindungen in einer Menge von 0,2 bis 0,6 Gew.-% bezogen auf das Gesamtgewicht der Zusammensetzung vorliegen.

**5.** Zusammensetzung nach einem der vorhergehenden Ansprüche, wobei die einen oder mehreren quartären Ammoniumverbindungen Didecyldimethylammoniumchlorid umfassen.

**6.** Zusammensetzung nach einem der vorhergehenden Ansprüche,

(i) wobei der aromatische Alkohol in einer Menge von 0,1 bis 0,5 Gew.-% bezogen auf das Gesamtgewicht der Zusammensetzung vorliegt, wobei die einen oder mehreren Tenside mindestens ein nichtionisches Tensid und mindestens ein amphoteres Tensid in einer Menge von 1,0 bis 4,0 Gew.-% bezogen auf das Gesamtgewicht der Zusammensetzung umfassen und wobei das Gewichtsverhältnis von amphoterem Tensid zu nichtionischem Tensid 1:1 bis 1:6 beträgt.

**7.** Zusammensetzung nach einem der vorhergehenden Ansprüche, wobei die einen oder mehreren quartären Ammoniumverbindungen Didecyldimethylammoniumchlorid sind.

**8.** Zusammensetzung nach einem der vorhergehenden Ansprüche, wobei die einen oder mehreren Tenside aus der Gruppe ausgewählt sind, die aus Polysorbat 20, Lauryldimethylaminoxid, Polyglyceryl-10-Caprylat/Caprat und Mischungen davon besteht.

**9.** Zusammensetzung nach einem der vorhergehenden Ansprüche, wobei die Zusammensetzung aufweist:

a. Didecyldimethylammoniumchlorid in einer Menge von 0,1 bis 0,7 Gew.-% bezogen auf das Gesamtgewicht der Zusammensetzung;
b. ein oder mehrere Tenside, ausgewählt aus Polysorbat 20, Lauryldimethylaminoxid und Polyglyceryl-10-Caprylat/Caprat, in einer Menge von 0,3 bis 3,0 Gew.% bezogen auf das Gesamtgewicht der Zusammensetzung; und

2-Phenoxyethanol in einer Menge von 0,1 bis 1,0 Gew.% bezogen auf das Gesamtgewicht der Zusammensetzung.

**10.** Zusammensetzung nach einem der vorhergehenden Ansprüche, ferner aufweisend ein Feuchthaltemittel, ein Lösungsmittel, ein Duftstoff, ein Färbemittel oder Farbstoff, ein Trübungsmittel, ein Perlglanzmittel, ein Vitamin, ein Antioxidans, einen Weichmacher, einen Hautpflegezusatz, ein Schaumbildungsmittel, einen polymeren Eindicker oder Mischungen davon.

**11.** Handseife in Form einer Flüssigkeit, eines Gels oder eines Schaums, die die Zusammensetzung nach einem der vorhergehenden Ansprüche aufweist.

## Revendications

**1.** Une composition désinfectante pour la peau comprenant:

a. un ou plusieurs composés d'ammonium quaternaire en une quantité allant de 0,1 à 2,0 % en poids par rapport au poids total de la composition, dans laquelle lesdits un ou plusieurs composés d'ammonium quaternaire comprennent un chlorure de diméthyldialkylammonium dans lequel chaque groupe alkyle contient 8 à 12 atomes de carbone;
b. un ou plusieurs tensioactifs en une quantité allant de 0,1 à 4,0 % en poids par rapport au poids total de la composition, dans laquelle lesdits un ou plusieurs tensioactifs sont choisis dans le groupe constitué par des tensioactifs non ioniques, des tensioactifs amphotères, et des mélanges de ceux-ci,

dans laquelle le tensioactif non ionique est choisi dans le groupe constitué par le caprylate/caprate de polyglycéryl-10, le polysorbate 20, le polysorbate 80, le decyl glucoside, l'éther laurylique de polyéthylène glycol (23), les éthers alkyliques d'hexaéthylène glycol en C9-11 et l'éther stéarylique de polyoxyéthylène (21),

et

dans lequel le tensioactif amphotère est choisi dans le groupe constitué par l'oxyde d'octyldiméthylamine, l'oxyde de décyldiméthylamine, l'oxyde de lauryldiméthylamine, l'oxyde d'isoalkyldiméthylamine, l'oxyde de tétradécyldiméthylamine, l'oxyde de cétyldiméthylamine, la bétaïne et le β-alanine de cocoamidopropyle, de N-(2-carboxyéthyl)-, N-coco alkyle dérivés; et

c. un alcool aromatique en une quantité allant de 0,1 à 1,0 % en poids par rapport au poids total de la composition, dans laquelle l'alcool aromatique est le phénoxyéthanol, et
dans laquelle la composition ne contient pas de tensioactif anionique.

**2.** La composition selon la revendication 1, dans laquelle l'alcool aromatique est présent en une quantité allant de 0,1 à 1,0 % en poids par rapport au poids total de la composition et dans laquelle le ou les tensioactifs comprennent au moins un tensioactif amphotère présent en une quantité allant de 0,1 et 4,0 % en poids, par rapport au poids total de la composition.

**3.** La composition selon l'une quelconque des revendications précédentes, dans laquelle le ou les tensioactifs comprennent au moins un tensioactif non ionique et au moins un tensioactif amphotère.

**4.** La composition selon l'une quelconque des revendications précédentes, dans laquelle le ou les composés d'ammonium quaternaire sont présents en une quantité allant de 0,2 à 1,0 % en poids par rapport au poids total de la composition ou dans laquelle le ou les composés d'ammonium quaternaire sont présents en une quantité allant de 0,2 à 0,6 % en poids par rapport au poids total de la composition.

**5.** La composition selon l'une quelconque des revendications précédentes, dans laquelle le ou les composés d'ammonium quaternaire comprennent du chlorure de didécyldiméthylammoniun.

**6.** La composition selon l'une quelconque des revendications précédentes,

(i) dans laquelle l'alcool aromatique est présent en une quantité allant de 0,1 à 0,5 % en poids par rapport au poids total de la composition, dans laquelle le ou les tensioactifs comprennent au moins un tensioactif non ionique et au moins un tensioactif amphotère en une quantité allant de 1,0 à 4,0 % en poids par rapport au poids total de la composition et dans laquelle le rapport pondéral du tensioactif amphotère au tensioactif non ionique est de 1: 1 à 1:6.

**7.** La composition selon l'une quelconque des revendications précédentes, dans laquelle le ou les composés d'ammonium quaternaire sont du chlorure de didécyldiméthylammonium.

**8.** La composition selon l'une quelconque des revendications précédentes, dans laquelle le ou les tensioactifs sont choisis dans le groupe constitué par le polysorbate 20, l'oxyde de lauryldiméthylamine, le caprylate/caprate de polyglycéryl-10 et leurs mélanges.

**9.** La composition selon l'une quelconque des revendications précédentes, dans laquelle la composition comprend

a. du chlorure de didécyldiméthylammonium en une quantité allant de 0,1 à 0,7.% en poids par rapport au poids total de la composition;
b. un ou plusieurs tensioactifs choisis parmi le polysorbate 20, l'oxyde de lauryl-diméthylamine, et le caprylate/caprate de polyglycéryl-10 en une quantité allant de 0,3 à 3,0 % en poids, par rapport au poids total de la composition; et

du 2-phénoxyéthanol en une quantité allant de 0,1 à 1,0 % en poids, par rapport au poids total de la composition.

**10.** La composition selon l'une quelconque des revendications précédentes, comprenant en outre un humectant, un solvant, un parfum, un agent colorant ou un colorant, un agent opacifiant, un agent nacrant, une vitamine, un antioxydant, un émollient, un additif de soin de la peau, un agent moussant, un épaississant polymère ou des mélanges de ceux-ci.

**11.** Un savon pour les mains sous forme de liquide, de gel ou de mousse comprenant la composition selon l'une quelconque des revendications précédentes.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017184614 A1
- WO 2013148247 A1
- US 20020022660 A
- US 20190125634 A
- US 20180153177 A
- CN 110897914
- CN 111096340
- WO 2013148247 A